# EUROPEAN PATENT APPLICATION

(11) **EP 4 706 650 A1**
(43) Date of publication of application: **11.03.2026**
(21) Application number: 24199460.7
(22) Date of filing: 10.09.2024
(51) Int. Cl.: A61K 31/167, A61K 31/4704, A61P 13/12

(54) **COMPOUNDS FOR USE IN THE TREATMENT OF KIDNEY DISEASES**

(71) Applicant: Universitätsmedizin Greifswald, 17475 Greifswald (DE)
(72) Inventor: Endlich, Nicole, 17493 Greifswald (DE); Schindler, Maximilian, 17489 Greifswald (DE)
(74) Representative: Cohausz & Florack

(57) **Abstract**

The present invention relates to a compound for use in the treatment and/or prevention of a kidney disease, wherein the compound has the general formula (I) or a salt, hydrate, solvate, metabolite, or prodrug thereof.

## Description

### FIELD OF THE INVENTION

The present invention relates to compounds for use in the treatment of a kidney disease. The compounds can be particularly used in the treatment and/or prevention of a kidney disease.

### BACKGROUND OF THE INVENTION

A global prevalence of more than 10% makes chronic kidney diseases a silent pandemic which causes severe and life-shortening problems for patients as well as the health care systems worldwide. In more than 75% of the kidney patients, a highly specific cell type in the kidney, the podocytes, is damaged or lost. Since podocytes are post-mitotic, there is no possibility of regeneration and lost podocytes are lost forever.

Podocytes cover the outer aspect of the capillaries in the filter unit of the kidney, the glomeruli, and are an essential part of the filtration barrier. Their tiny cell processes, so called foot processes (FP), interdigitate in a very regular and zipper-like manner. Between the interdigitating foot processes, a slit membrane is spanned which is also essential for the size selectivity of the filtration barrier (Pavenstadt, H. et al. "Cell biology of the glomerular podocyte." Physiological reviews 83.1 (2003): 253-307; Garg, Puneet. "A review of podocyte biology." American journal of nephrology 47.1 (2018): 3-13). Injury of FP or detachment of podocytes leads to proteinuria, which is a clinical hallmark of kidney disease and characterized by the loss of high molecular weight proteins.

Such a damage to podocyte foot processes often leads to end-stage kidney disease (ESKD, Shankland, S. J. "The podocyte's response to injury: role in proteinuria and glomerulosclerosis." Kidney international 69.12 (2006): 2131-2147; Kriz, Wilhelm. "Podocyte hypertrophy mismatch and glomerular disease." Nature Reviews Nephrology 8.11 (2012): 618-619) and finally to renal failure making dialysis and transplantation necessary to survive. One specific podocytopathy is the focal and segmental glomerulosclerosis (FSGS). FSGS is characterized by the effacement of FP, the loss of podocytes, matrix accumulation, activated parietal epithelial cell (PECs) of the Bowman's capsule and sclerotic lesions in the glomeruli. Since many kidney disease are painless, kidney diseases, in particular glomerulopathies and/or podocyte-injury-related diseases, are diagnosed very late. Moreover, no healing drugs or therapies are available. Thus, there is a need for improved methods to treat and prevent kidney diseases.

### SUMMARY OF THE INVENTION

Against the aforementioned background, it is an object of the present invention to provide improved methods of treatment and prevention of kidney diseases, in particular to provide safe and effective methods for treatment and prevention of kidney diseases including in particular acute and chronic kidney diseases.

These objects are achieved by the compounds for use of claim 1.

Claim 1 concerns compounds for use in the treatment and/or prevention of a kidney disease, wherein the compounds have the general formula (I): 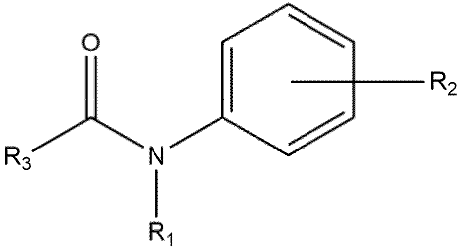 wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl and C₁-C₃-alkoxy;
R₂ is selected from the group consisting of -NH₂, C₁-C₃-haloalkyl, C₁-C₃-alkyl, C₁-C₃-alkoxy, C₁-C₃-haloalkoxy, C₃-C₆-cycloalkyl, halogen, -OH, -SH, -CN, -CHO, -NH(C₁-C₃-alkyl) and -N(C₁-C₃-alkyl)₂;
R₃ is selected from the group consisting of 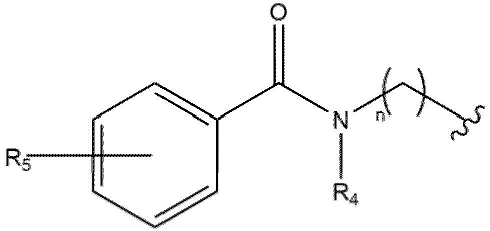 and 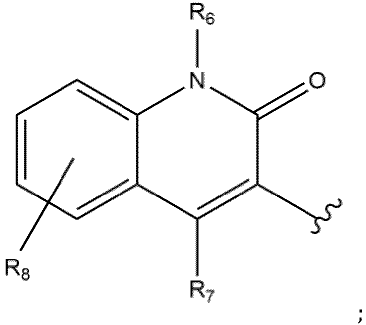
R₄ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
n is 2, 3, 4, 5, 6, or 7;
R₅ is selected from the group consisting of C₁-C₃-alkyl, C₁-C₃-haloalkyl, C₁-C₃-alkoxy, C₃-C₆-cycloalkyl, C₂-C₄-alkenyl, C₂-C₄-alkynyl, halogen, -OH, -SH, -CN, -CHO, -COOH, -NH₂, -CONH₂, -CSNH₂, -NH(C₁-C₃-alkyl), and -N(C₁-C₃-alkyl)₂;
R₆ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₇ is selected from the group consisting of -OH, -CN, -SH, -CHO, COOH, -NH₂, -NH(C₁-C₃-alkyl), -N(C₁-C₃alkyl)₂, C₁-C₃-alkoxy, -O-C=O-C₁-C₃alkyl, and C₁-C₃-hydroxyalkyl; and
R₈ is selected from the group constisting of C₁-C₃-alkoxy, C₁-C₃-alkyl, C₁-C₃-hydroxyalkyl, C₁-C₃-haloalkyl, C₂-C₄-alkenyl, C₂-C₄-alkynyl, -OH, -SH, -CN, -CHO, -COOH, NH₂, -CONH₂, - CSNH₂, -NH(C₁-C₃-alkyl), and -N(C₁-C₃alkyl)₂;
or a salt, hydrate, solvate, metabolite, or prodrug thereof.

The inventors found that compounds of formula (I) were particularly effective in a well-established animal model of focal and segmental glomerulosclerosis (FSGS), a kidney disease and in particular a disease related to podocyte injury.

By employing the compounds of formula (I) (or salts, solvates, hydrates, metabolites, or prodrugs thereof) in the treatment of kidney diseases, in particular of chronic or acute kidney diseases, the inventors provide a greatly needed means to safely and effectively treat and prevent podocyte damage, apoptosis, or loss, which is a hallmark of kidney diseases, in particular of podocyte injury-related diseases such as FSGS and minimal change disease.

In particular, the inventors found that in the well-established zebrafish larvae model of FSGS, where apoptosis of podocytes is induced in a gradual and specific manner (Hansen, Kerrin Ursula Ingeborg, et al. "Prolonged podocyte depletion in larval zebrafish resembles mammalian focal and segmental glomerulosclerosis." The FASEB Journal 34.12 (2020): 15961-15974), test compounds including 1,2-Dihydro-4-hydroxy-5-methoxy-N,1-dimethyl-2-oxo-N-[4-(trifluoromethyl)phenyl]-3-quinolinecarboxamide (also known as Tasquinimod, CAS. Nr.; 254964-60-8), and N-(6-((2-aminophenyl)amino)-6-oxohexyl)-4-methylbenzamide (also known as RG2833, CAS. Nr. : 1215493-56-3), significantly slowed the progression of podocyte injury induced in the disease model and even improved disease-related symptoms. The effects occurred in a concentration-dependent manner.

Thus, the invention provides safe and effective compounds for use in treating and/or preventing kidney diseases, in particular of kidney diseases such as glomerulopathies and/or podocyte injury-related diseases such as FSGS and minimal change disease.

### DETAILED DESCRIPTION OF ILLUSTRATIVE EMBODIMENTS

The invention provides compounds of formula (I) for use in the treatment and/or prevention of a kidney disease.

The inventors found that compounds with the shared general structure of formula (I), as described above had a protective effect on podocytes in a validated injury model of focal and segmental glomerulosclerosis (FSGS). This FSGS model is also representative of other kidney diseases, in particular acute and chronic kidney diseases, including but not limited to other glomerulopathies and podocyte-injury-related diseases.

### Compounds according to alternative I

In one embodiment, the compound for use in the treatment and/or prevention of a kidney disease according to alternative (I) has the general formula (I): 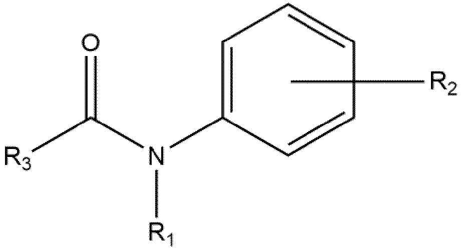 wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₂ is selected from the group consisting of -NH₂, C₁-C₃-haloalkyl, C₁-C₃-alkyl, C₁-C₃-alkoxy, C₁-C₃-haloalkoxy, C₃-C₆-cycloalkyl, halogen, -OH, -SH, -CN, -CHO, -NH(C₁-C₃-alkyl), and -N(C₁-C₃-alkyl)₂;
R₃ is 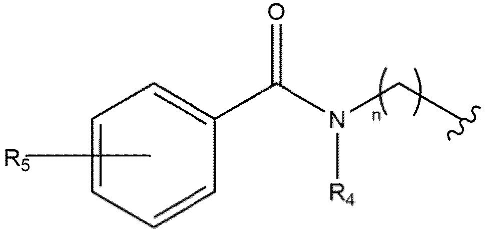
R₄ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
n is 2, 3, 4, 5, 6, or 7; and
R₅ is selected from the group consisting of C₁-C₃-alkyl, C₁-C₃-haloalkyl, C₁-C₃-alkoxy, C₃-C₆-cycloalkyl, C₂-C₄-alkenyl, C₂-C₄-alkynyl, halogen, -OH, -SH, -CN, -CHO, -COOH, -NH₂, -CONH₂, -CSNH₂, -NH(C₁-C₃-alkyl), and -N(C₁-C₃-alkyl)₂;
or a salt, hydrate, solvate, metabolite, or prodrug thereof.

In one embodiment, the compound for use in the treatment and/or prevention of a kidney disease according to alternative (I) is a compound of formula (I),
wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₂ is selected from the group consisting of -NH₂, C₁-C₃-haloalkyl, C₁-C₃-alkoxy, C₁-C₃-haloalkoxy, halogen, -OH, -SH, -CN, -CHO, -NH(C₁-C₃-alkyl), and -N(C₁-C₃alkyl)₂;
R₃ is 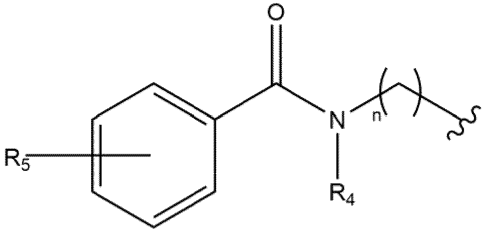
R₄ is selected from the group consisting of hydrogen, C₁-C₃-alkyl, C₁-C₃-haloalkyl, C₃-C₆-cycloalkyl, and C₁-C₃-alkoxy;
n is 4, 5, or 6, preferably 5; and
R₅ is selected from the group consisting of C₁-C₃-alkyl, C₁-C₃-haloalkyl, C₁-C₃-alkoxy, C₃-C₆-cycloalkyl, C₂-C₄-alkenyl, C₂-C₄-alkynyl, halogen, -OH, -SH, -CN, -CHO, -COOH, -NH₂, -CONH₂, -CSNH₂, -NH(C₁-C₃-alkyl), and -N(C₁-C₃alkyl)₂;
or a salt, hydrate, solvate, metabolite, or prodrug thereof.

In one embodiment, the compound for use in the treatment and/or prevention of a kidney disease according to alternative (I) is a compound of formula (I),
wherein
R₁ is selected from the group consisting of hydrogen, C₁-C₃-alkyl, and C₃-C₆-cycloalkyl;
R₂ is selected from the group consisting of -NH₂, C₁-C₃-haloalkyl, C₁-C₃-alkoxy, halogen, -OH, -SH, -CN, -CHO, -NH(C₁-C₃-alkyl), and -N(C₁-C₃alkyl)₂;
R₄ is selected from the group consisting of hydrogen, C₁-C₃-alkyl, and C₃-C₆-cycloalkyl;
n is 5; and
R₅ is selected from the group consisting of C₁-C₃-alkyl, C₁-C₃-haloalkyl, C₁-C₃-alkoxy, C₃-C₆-cycloalkyl, C₂-C₄-alkenyl, and C₂-C₄-alkynyl;
or a salt, hydrate, solvate, metabolite, or prodrug thereof.

In one embodiment, the compound for use in the treatment and/or prevention of a kidney disease according to alternative (I) is a compound of formula (I),
wherein
R₁ is selected from the group consisting of hydrogen, methyl, ethyl, cyclopropyl, and cyclobutyl;
R₂ is selected from the group consisting of -NH₂, -CF₃, -CCl₃, -CBr₃, -CI₃, -CHF₂, -CHCl₂, -CHBr₂, -CHCl₂, -CH₂F, -CHzCl, -CH₂Br, -CH₂I, trifluormethoxy, trichlormethoxy, tribromomethoxy, trijodomethoxy, difluormethoxy, dichlormethoxy, dibromomethoxy, dijodomethoxy, fluormethoxy, chlormethoxy, bromomethoxy, jodomethoxy, -CH₂CF₃, -CH₂CCl₃, -CH₂CBr₃, -CH₂CI₃, -CH₂CHF₂, -CH₂CHCl₂, -CH₂CHBr₂, -CH₂CHCl₂, -CH₂CH₂F, -CHzCHzCl, -CHzBr, -CH₂CH₂I, trifluorethoxy, trichlorethoxy, tribromoethoxy, trijodoethoxy, difluorethoxy, dichlorethoxy, dibromoethoxy, dijodoethoxy, fluorethoxy, chlorethoxy, bromoethoxy, jodoethoxy, methoxy, ethoxy, -F, -Br, -Cl, -I, -OH, -SH, -CN, -CHO, -NH(CH₃), -NH(CH₂CH₃), -N(CH₂CH₃)₂, and -N(CH₃)₂;
R₄ is selected from the group consisting of hydrogen, methyl, ethyl, propyl, and butyl;
n is 5; and
R₅ is selected from the group consisting of methyl, ethyl, -CF₃, -CCl₃, -CBr₃, -CI₃, -CHF₂, -CHCl₂, -CHBr₂, -CHCl₂, -CH₂F, -CH₂Cl, -CHzBr, -CH₂I, methoxy, ethoxy, cyclopropyl, cyclobutyl, ethenyl, and ethinyl;
or a salt, hydrate, solvate, metabolite, or prodrug thereof.

In one embodiment, the compound for use in the treatment and/or prevention of a kidney disease according to alternative (I) is a compound of formula (I),
wherein
R₁ is selected from the group consisting of hydrogen, methyl, ethyl, cyclopropyl, and cyclobutyl;
R₂ is selected from the group consisting of -NH₂, -CF₃, -CCl₃, -CBr₃, -CI₃, -CHF₂, -CHCl₂, -CHBr₂, -CHCl₂, -CH₂F, -CHzCl, -CH₂Br, -CH₂I, trifluormethoxy, trichlormethoxy, tribromomethoxy, trijodomethoxy, difluormethoxy, dichlormethoxy, dibromomethoxy, dijodomethoxy, fluormethoxy, chlormethoxy, bromomethoxy, jodomethoxy, methoxy, ethoxy, -F, -Br, -Cl, -I, -OH, -SH, -CN, -CHO, -NH(CH₃), and -N(CH₃)₂;
R₄ is selected from the group consisting of hydrogen, methyl, ethyl, propyl, and butyl;
n is 5; and
R₅ is selected from the group consisting of methyl, ethyl, cyclopropyl, and cyclobutyl; or a salt, hydrate, solvate, metabolite, or prodrug thereof.

In one embodiment, the compound for use in the treatment and/or prevention of a kidney disease according to alternative (I) is a compound of formula (I),
wherein the compound is 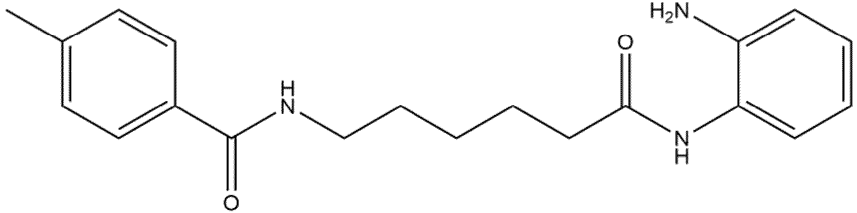
or a salt, hydrate, solvate, metabolite, or prodrug thereof. This compound (N-(6-((2-aminophenyl)amino)-6-oxohexyl)-4-methylbenzamide) is also know as RG2833. It is a known histone deacetylase inhibitor. The molecular formula is C₂₀H₂₅N₃O₂ and the molecular weight is 339.43 g/mol.

In one embodiment, the compound for use in the treatment and/or prevention of a kidney disease according to alternative (I) is a compound of formula (I),
wherein the compound is 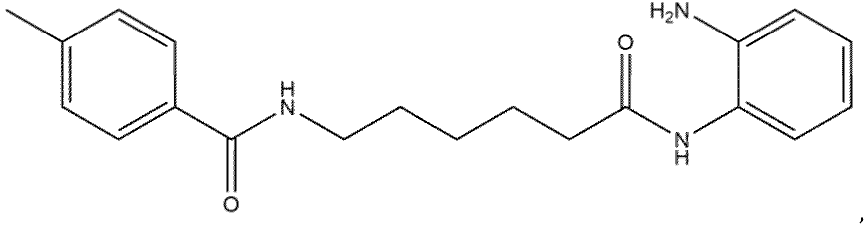
or a salt, hydrate, solvate, metabolite, or prodrug thereof,
wherein the metabolite is selected from the group consisting of a benzimidazole, a glucuronide and products of amide hydrolysis including o-phenylenediamine, acetylated phenylenediamine and acids.

The compound for use according to alternative (I) may also be a pharmaceutically-acceptable salt, solvate, or hydrate. As used herein, the terms "salt" and "pharmaceutically acceptable salt" are used interchangeably. Examples of pharmaceutically acceptable salts are well known in the art and are for example discussed in Berge et at., 1977, "Pharmaceutically Acceptable Salts." J. Pharm. ScL. Vol. 66, pp. 1-19. In some embodiments, the pharmaceutically-acceptable salt is selected from the group of inorganic cations, organic cations, inorganic anions, organic anions, and polymeric organic anions.

Examples of suitable inorganic cations include, but are not limited to, alkali metal ions such as Na⁺ and K+ , alkaline earth cations such as Ca²⁺ and Mg²⁺, and other cations such as Al³⁺. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric.

Examples of suitable polymeric organic anions include, but are not limited to, those derived from the following polymeric acids: tannic acid, carboxymethyl cellulose.

Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N- methylglucamine, oxalate, pamoate (embonate), palmitate, pantothenate, phosphate/diphosphate, polygalacturonate, potassium, salicylate, sodium, stearate, sub acetate, succinate, tannate, tartrate, teoclate, tosylate, triethiodide, trimethylammonium, and valerate.

The terms "solvate" and "hydrate" have their normal meaning In the art. The term "solvate", for example, is used herein in the conventional sense to refer to a complex of solute and solvent. If the solvent is water, the solvate may be conveniently referred to as a hydrate, for example, a mono-hydrate, a di-hydrate, a tri-hydrate.

The compound for use in the treatment and/or prevention of a kidney disease according to alternative (I) in some embodiments is a produg. A prodrug has its normal meaning in the art and refers to a pharmacologically inactive drug precursor that is converted into a pharmacologically active drug compound.

### Compounds according to alternative II

In one embodiment, the compound for use in the treatment and/or prevention of a kidney disease according to alternative (II) has the general formula (I): 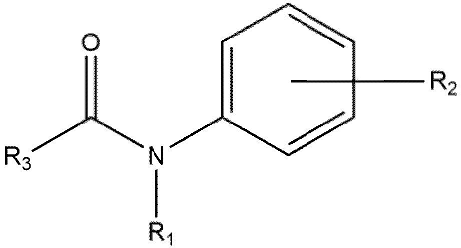 wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₂ is selected from the group consisting of -NH₂, C₁-C₃-haloalkyl, preferably -CF₃, C₁-C₃-alkyl,
C₁-C₃-alkoxy, C₁-C₃-haloalkoxy, C₃-C₆-cycloalkyl, halogen, -OH, -SH, -CN, -CHO, -NH(C₁-C₃-alkyl), and -N(C₁-C₃-alkyl)₂;
R₃ is 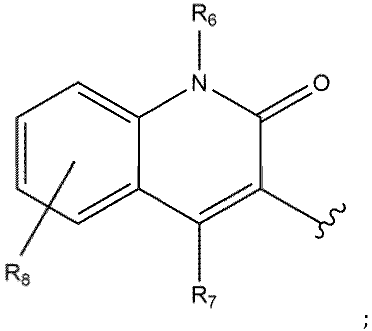
R₆ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₇ is selected from the group consisting of -OH, -CN, -SH, -CHO, COOH, -NH₂, -NH(C₁-C₃-alkyl), -N(C₁-C₃alkyl)₂, C₁-C₃-alkoxy, -O-C=O-C₁-C₃alkyl, and C₁-C₃-hydroxyalkyl; and
R₈ is selected from the group constisting of C₁-C₃-alkoxy, C₁-C₃-alkyl, C₁-C₃-hydroxyalkyl, C₁-C₃-haloalkyl, C₂-C₄-alkenyl, C₂-C₄-alkynyl, -OH, -SH, -CN, -CHO, -COOH, NH₂, -CONH₂, -CSNH₂, -NH(C₁-C₃-alkyl), and -N(C₁-C₃alkyl)₂;
or a salt, hydrate, solvate, metabolite, or prodrug thereof.

In one embodiment, the compound for use in the treatment and/or prevention of a kidney disease according to alternative (II) is a compound of formula (I),
wherein
R₁ is selected from the group consisting of hydrogen, C₁-C₃-alkyl, and C₃-C₆-cycloalkyl;
R₂ is selected from the group consisting of -NH₂, C₁-C₃-haloalkyl, preferably -CF₃, C₁-C₃-alkyl, C₁-C₃-alkoxy, C₁-C₃-haloalkoxy, C₃-C₆-cycloalkyl, halogen, -OH, -SH, -CN, -CHO, -NH(C₁-C₃-alkyl), and -N(C₁-C₃alkyl)₂;
R₃ is 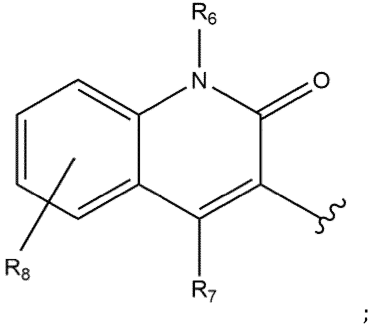
R₆ is selected from the group consisting of hydrogen, C₁-C₃-alkyl, C₁-C₃-haloalkyl, C₃-C₆-cycloalkyl, and C₁-C₃-alkoxy;
R₇ is selected from the group consisting of -OH, -CN, -SH, -CHO, COOH, -NH₂, -NH(C₁-C₂-alkyl), -N(C₁-C₂-alkyl)₂, C₁-C₂-alkoxy, -O-C=O-C₁-C₃alkyl, and C₁-C₂-hydroxyalkyl; and
R₈ is selected from the group consisting of C₁-C₃-alkoxy, C₁-C₃-alkyl, C₁-C₃-hydroxyalkyl, C₁-C₃-haloalkyl, -OH, -SH, -CN, -CHO, -COOH, NH₂, -CONH₂, -CSNH₂, -NH(C₁-C₃-alkyl), and -N(C₁-C₃alkyl)₂,
or a salt, hydrate, solvate, metabolite, or prodrug thereof.

In one embodiment, the compound for use in the treatment and/or prevention of a kidney disease according to alternative (II) is a compound of formula (I),
wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₂ is selected from the group consisting of -NH₂, C₁-C₃-haloalkyl, preferably -CF₃, C₁-C₃-alkyl, C₁-C₃-alkoxy, C₃-C₆-cycloalkyl, halogen, -OH, -SH, -CN, -CHO, -NH(C₁-C₃-alkyl), and -N(C₁-C₃alkyl)₂;
R₃ is 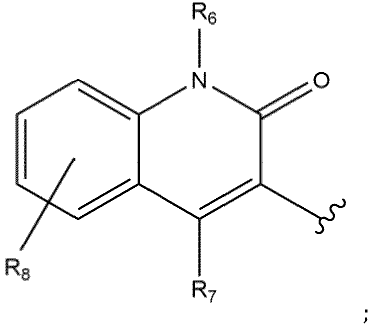
R₆ is selected from the group consisting of hydrogen, C₁-C₃-alkyl, and C₃-C₆-cycloalkyl;
R₇ is selected from the group consisting of -OH, -CN, -SH, -CHO, COOH, -NH₂, -NH-CH₃, -N(CH₃)₂, -OCH₃, -O-C=O-CH₂-CH₃, and -CH₂-OH; and
R₈ is selected from the group consisting of C₁-C₂-alkoxy, C₁-C₂-alkyl, C₁-C₂-hydroxyalkyl, C₁-C₂-haloalkyl, -OH, -SH, -CN, -CHO, -COOH, -NH₂, -CONH₂, -CSNHz, -NH(C₁-C₂-alkyl), and -N(C₁-C₂-alkyl)₂,
or a salt, hydrate, solvate, metabolite, or prodrug thereof.

In one embodiment, the compound for use in the treatment and/or prevention of a kidney disease according to alternative (II) is a compound of formula (I),
wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₂ is selected from the group consisting of -NH₂, C₁-C₃-haloalkyl, preferably -CF₃, C₁-C₃-alkyl, C₁-C₃-alkoxy, C₃-C₆-cycloalkyl, halogen, -OH, -SH, -CN, -CHO, -NH(C₁-C₃-alkyl), and -N(C₁-C₃alkyl)₂;
Ra is 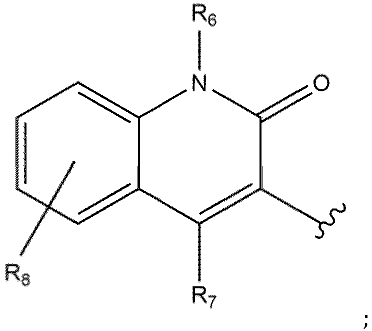
R₆ is selected from the group consisting of hydrogen, C₁-C₃-alkyl, and C₃-C₆-cycloalkyl;
R₇ is selected from the group consisting of -OH, -CN, -SH, -CHO, COOH, -NH₂, -NH-CH₃, -N(CH₃)₂, -OCH₃, -O-C=O-CH₂-CH₃, and -CH₂-OH; and
R₈ is selected from the group consisting of C₁-C₂-alkoxy, C₁-C₂-alkyl, C₁-C₂-hydroxyalkyl, C₁-C₂-haloalkyl, -OH, -SH, -CN, -CHO, -COOH, -NH₂, -CONH₂, -CSNHz, -NH(C₁-C₂-alkyl) and -N(C₁-C₂-alkyl)₂,
or a salt, hydrate, solvate, metabolite, or prodrug thereof.

In one embodiment, the compound for use in the treatment and/or prevention of a kidney disease according to alternative (II) is a compound of formula (I),
wherein
R₁ is selected from the group consisting of hydrogen, methyl, ethyl, cyclopropyl, cyclobutyl, ethenyl, and ethinyl;
R₂ is selected from the group consisting of -NH₂, -CF₃, -CCl₃, -CBr₃, -CI₃, -CHF₂, -CHCl₂, -CHBr₂, -CHCl₂, -CH₂F, -CHzCl, -CHzBr, -CH₂I, trifluormethoxy, trichlormethoxy, tribromomethoxy, trijodomethoxy, difluormethoxy, dichlormethoxy, dibromomethoxy, dijodomethoxy, fluormethoxy, chlormethoxy, bromomethoxy, jodomethoxy, -CH₂CF₃, -CH₂CCl₃, -CH₂CBr₃, -CH₂CI₃, -CH₂CHF₂, -CH₂CHCl₂, -CH₂CHBr₂, -CH₂CHCl₂, -CH₂CH₂F, -CHzCHzCl, -CHzBr, -CH₂CH₂I, trifluorethoxy, trichlorethoxy, tribromoethoxy, trijodoethoxy, difluorethoxy, dichlorethoxy, dibromoethoxy, dijodoethoxy, fluorethoxy, chlorethoxy, bromoethoxy, jodoethoxy, methoxy, ethoxy, -F, -Br, -Cl, -I, -OH, -SH, -CN, -CHO, -NH(CH₃), -NH(CH₂CH₃), -N(CH₂CH₃)₂ and -N(CH₃)₂;
R₃ is 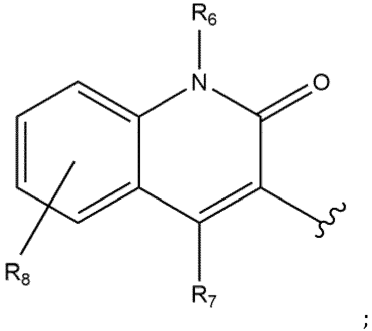
R₆ is selected from the group consisting of hydrogen, methyl, ethyl, cyclopropyl, and cyclobutyl;
R₇ is selected from the group consisting of -OH, -CN, -SH, -CHO, -COOH, -NH₂, -NH-CH₃, -N(CH₃)₂, -OCH₃, -O-C=O-CH₂-CH₃, and -CH₂-OH; and
R₈ is selected from the group consisting of methoxy, methyl, hydroxymethyl, -CF₃, -CCl₃, - CBr₃, -CI₃, -CHF₂, -CHCl₂, -CHBr₂, -CHCl₂, -CH₂F, -CH₂Cl, -CH₂Br, -CH₂I, -OH, -SH, -CN, -CHO, -COOH, -NH₂, -CONH₂, -CSNH₂, -NH(C₁-C₂-alkyl), and -N(C₁-C₂-alkyl)₂,
or a salt, hydrate, solvate, metabolite, or prodrug thereof.

In one embodiment, the compound for use in the treatment and/or prevention of a kidney disease according to alternative (II) is a compound of formula (I),
wherein
R₁ is selected from the group consisting of hydrogen, methyl, ethyl, cyclopropyl, and cyclobutyl;
R₂ is selected from the group consisting of -NH₂, -CF₃, -CCl₃, -CBr₃, -CI₃, trifluormethoxy, trichlormethoxy, tribromomethoxy, trijodomethoxy, -F, -Br, -Cl, -I, -OH, -SH, -CN, -CHO, -NH(CH₃), -NH(CH₂CH₃), -N(CH₂CH₃)₂ and -N(CH₃)₂;
R₃ is 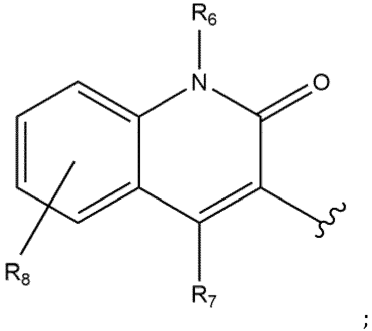
R₆ is selected from the group consisting of hydrogen, methyl, and ethyl;
R₇ is selected from the group consisting of -OH, -CN, -SH, -CHO, -COOH, -NH₂, -NH-CH₃, -N(CH₃)₂, -OCH₃, -O-C=O-CH₂-CH₃, and -CH₂-OH; and
R₈ is selected from the group consisting of methoxy, methyl, hydroxymethyl, -CF₃, -CCl₃, -CBr₃, -CI₃, -CHF₂, -CHCl₂, -CHBr₂, -CHCl₂, -CH₂F, -CHzCl, -CH₂Br, -CH₂I, -OH, -SH, -CN, -CHO, -COOH, -NH₂, -CONH₂, and -CSNH₂;
or a salt, hydrate, solvate, metabolite, or prodrug thereof.

In one embodiment, the compound for use in the treatment and/or prevention of a kidney disease according to alternative (II) is a compound of formula (I),
wherein the compound is 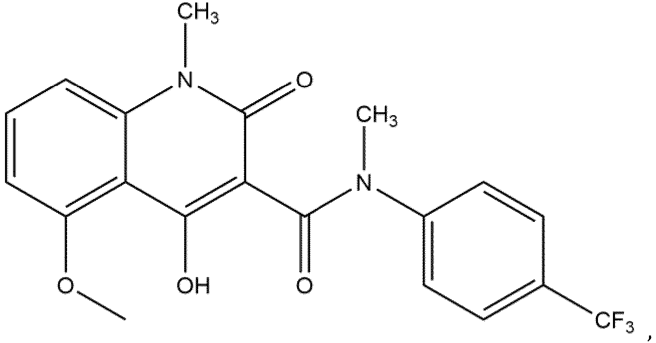
or a salt, hydrate, solvate, metabolite, or prodrug thereof. This compound (4-hydroxy-5-methoxy-N,1-dimethyl-2-oxo-N-(4-(trifluoromethyl)phenyl)-1,2-dihydroquinoline-3-carboxamide) is also known as Tasquinimod. It is a known histone deacetylase inhibitor. The molecular formula is C₂₀H₁₇F₃N₂O₄ and the molecular weight is 406.36 g/mol.

In one embodiment, the compound for use in the treatment and/or prevention of a kidney disease according to alternative (II) is a compound of formula (I),
wherein the compound is 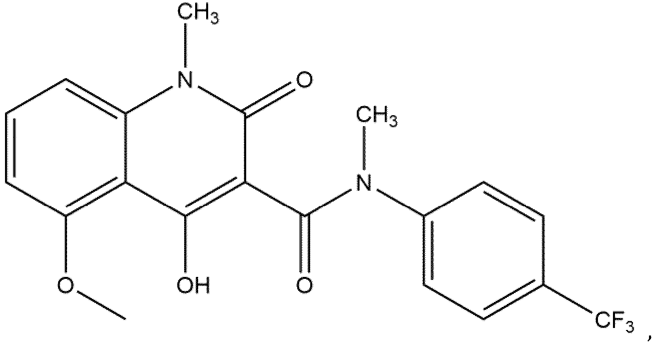
or a salt, hydrate, solvate, metabolite, or prodrug thereof,
wherein the metabolite is selected from the group consisting of

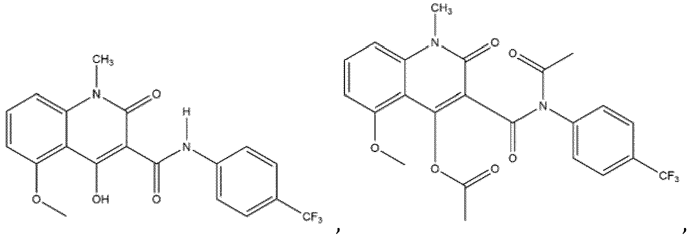

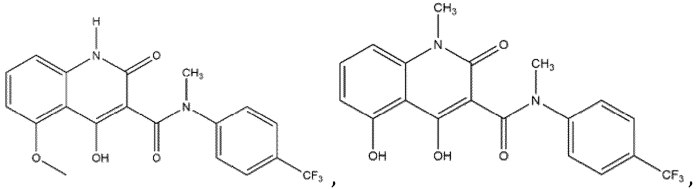

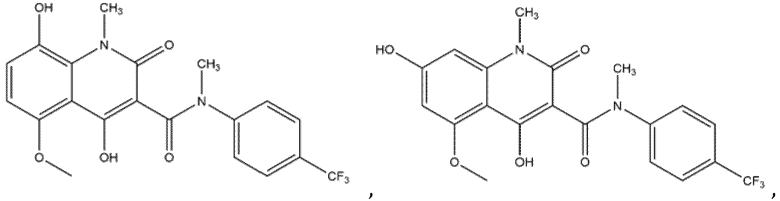

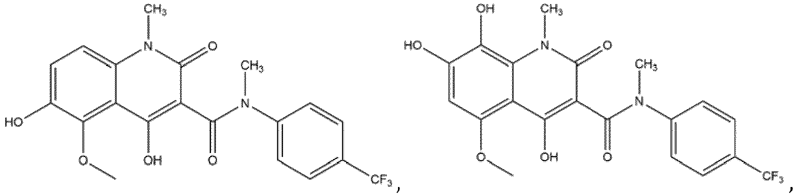

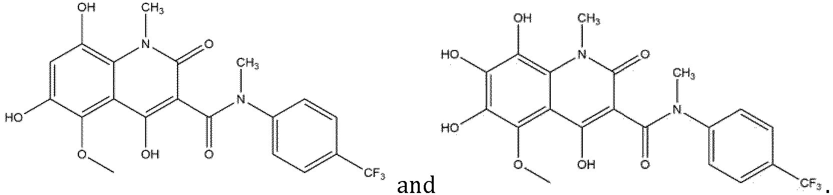

The compound for use according to alternative (II) may also be a pharmaceutically-acceptable salt, solvate, or hydrate. As used herein, the terms "salt" and "pharmaceutically acceptable salt" are used interchangeably. Examples of pharmaceutically acceptable salts are well known in the art and are for example discussed in Berge et at., 1977, "Pharmaceutically Acceptable Salts." J. Pharm. ScL. Vol. 66, pp. 1-19. In some embodiments, the pharmaceutically-acceptable salt is selected from the group of inorganic cations, organic cations, inorganic anions, organic anions, and polymeric organic anions.

Examples of suitable inorganic cations include, but are not limited to, alkali metal ions such as Na⁺ and K+, alkaline earth cations such as Ca²⁺ and Mg²⁺ , and other cations such as Al³⁺. Examples of suitable organic cations include, but are not limited to, ammonium ion (i.e., NH₄⁺) and substituted ammonium ions (e.g., NH₃R⁺, NH₂R₂⁺, NHR₃⁺, NR₄⁺). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric.

Examples of suitable polymeric organic anions include, but are not limited to, those derived from the following polymeric acids: tannic acid, carboxymethyl cellulose.

Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, sub acetate, succinate, tannate, tartrate, teoclate, tosylate, triethiodide, trimethylammonium, and valerate.

The terms "solvate" and "hydrate" have their normal meaning In the art. The term "solvate", for example, is used herein in the conventional sense to refer to a complex of solute and solvent. If the solvent is water, the solvate may be conveniently referred to as a hydrate, for example, a mono-hydrate, a di-hydrate, a tri-hydrate.

The compound for use in the treatment and/or prevention of a kidney disease according to alternative (II) in some embodiments is a produg. A prodrug has its normal meaning in the art and refers to a pharmacologically inactive drug precursor that is converted into a pharmacologically active drug compound.

### Treatment

It is contemplated that the present invention is relevant to the treatment of a broad range of diseases affecting the kidney. In some embodiments, the compounds of the invention are used to treat chronic and/or acute kidney disease.

In one embodiment, the chronic kidney disease is selected from the group consisting of primary or secondary kidney disease. The term primary kidney disease refers to a kidney disease that has its origin in the kidney. Primary kidney diseases can be grouped into various categories, such as glomerulonephritis, tubulointerstitial disease, genetic kidney disease, and kidney cancer. In one embodiment of the invention, the compounds are used for the treatment and/or prevention of a primary kidney disease selected from the group consisting of glomerulonephritis, tubulointerstitial disease, genetic kidney disease, and kidney cancer. In a preferred embodiment of the invention, the compounds are used for the treatment and/or prevention of a primary kidney disease selected from the group consisting of glomerulonephritis, tubulointerstitial disease, and genetic kidney disease; most preferably glomerulonephritis.

Preferably, the glomerulonephritis is selected from the group consisting of primary focal and segmental glomerulosclerosis (also referred to herein as primary "FSGS"), genetic FSGS (for example FSGS caused by one or more mutations of one or more of the 60 known genes such as NPHS1, NPHS2, WT1, MAG12, and/or APOL1, preferably APOL1), Alport syndrome, Fabry disease, Lupus nephritis, minimal change nephropathy, membranous glomerulonephritis, IgA glomerulonephritis, IgM glomerulonephritis, membranoproliferative glomerulonephritis, crescentic glomerulopathies (acute and chronic), age-related glomerulopathy (for example due to the loss of nephrons and/or podocytes), and cystic kidney disease. In a particularly preferred embodiment, the compounds are used in the treatment and/or prevention of a glomerulonephritis selected from the group consisting of primary FSGS, genetic FSGS, minimal change nephropathy, and age-related glomerulopathy.

Preferably, the tubulointerstitial disease is selected from the group consisting of interstitial nephritis, tubular acidosis, kidney stone-induced tubulointerstitial disease, urinary-tract infection-induced tubulointerstitial disease, and nephronophthisis. Preferably, the genetic kidney disease is a polycystic kidney disease.

The term secondary kidney disease refers to a kidney disease that has a systemic origin and did not originate in the kidney. Preferably the secondary kidney disease is selected from the group consisting of secondary FSGS, hypertension-induced nephropathy, diabetic nephropathy, minimal change disease, membranous nephropathy, a virus-induced kidney disease (for example HIV, SARS-CoV2, malaria, hepatitis-induced kidney disease), a systemic autoimmune disease (for example systemic lupus erythematosus), a drug-induced kidney disease (for example a NRSA, antibiotic, lithium, or inorganic salt such as gold and mercury-induced kidney disease), a tumor-induced kidney disease (for example a lung and/or colon tumor-induced kidney disease), immune complex-induced kidney disease, in the blood circulating antibody against e.g. phospholipase A₂ receptor (PLA2R), THSD7A, NELL-1, EXT1/2, Semaphorin 3B, HTRA1, PCDH7, a crescentic glomerulopathy, a toxin-induced kidney disease, a vascular and/or heart disease-induced kidney disease, and cystinosis. In a particularly preferred embodiment, the kidney disease is selected from the group consisting of secondary FSGS, hypertension-induced nephropathy, diabetic nephropathy, minimal change disease, and membranous nephropathy. Most preferably, the kidney disease is selected from the group consisting of secondary FSGS and minimal change disease.

The kidney disease treated according to the invention is not associated with or the result of a peripheral T-cell lymphoma. The treatment according to the invention does not comprise the treatment of a peripheral T-cell lymphoma.

In some embodiments, the kidney disease is an acute kidney injury, preferably selected from the group consisting of an infection-induced acute kidney injury (for example a bacteria or virus-induced kidney injury), dehydration-induced acute kidney injury, drug- or drug treatment-induced acute kidney injury (such as antibiotic-induced kidney injury), an obstruction of urine flow-induced acute kidney injury (for example from one or more kidney stones), a surgery-induced acute kidney injury, and necrosis-induced acute kidney injury.

In some embodiments, the kidney disease is a kidney disease associated with podocyte injury and/or podocyte foot process effacement. In a preferred embodiment, the kidney disease is a podocytopathy; in particular a podocytopathy selected from the group consisting of diffuse mesangial sclerosis, focal segmental glomerulosclerosis, minimal change disease, and collapsing glomerulopathy. In another preferred embodiment, the kidney disease is a genetic kidney disease associated with one or more genes located in the podocyte, preferably selected from the group consisting of COQ2/6, PDSS2, ADCK4, MTT1, MT-ATP6, PTPRO, EMP2, APOL1, PODXL, SCARB2, ZMPSTE24, PMM2, DGKE, GAPVD1, ANKFY1, SPGL1, TBC1D8B, CLCN5, ACTN4, MYH9, INF2, MYOE1, MAGI2, TNS2, DLC1, CDK20, ITSN1, ITSN2, ANLN, ARGHGAP24, ARGH-DIA, KANK1/2/4, SYNPO, AVIL, KAT2B, KEOPS-complex, WT1, LMX1B, SMARCAL1, NUP85/93/107/133/160/205, XPO5, E2F3, NXF5, PAX2, LMNA, MAFB, WDR73, NPHS1/2, PLCE1, CD2AP, TRPC6, CRB2, FAT1, and/or KIRREL1/2; in particular one or more genes selected from the group consisting of NPHS1/2, WT1, MAGI2, and/or APOL1.

As used herein, "treatment" means any manner in which one or more symptoms associated with a disease are beneficially altered. Accordingly, the term includes healing or amelioration of a symptom or side effect of the disease or a decrease in the rate of advancement of the disorder.

The term "subject" is intended to include humans, primates, livestock animals (e.g. horses, camels, cattle, sheep, pigs and donkeys), laboratory test animals (e.g. mice, rats, rabbits, guinea pigs), companion animals (e.g. dogs, cats), captive wild animals (e.g. kangaroos, deer, foxes), poultry birds (e.g. chickens, ducks, bantams, pheasants), reptiles, and fish. In a preferred embodiment, the subject is a human or a laboratory test animal. In a particularly preferred embodiment the subject is a human.

### Formulation, Dosage, and Administration

The compounds of the present invention can be formulated as a pharmaceutical composition and can be administered in any suitable manner. Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

The formulation comprises the compound of interest and may optionally comprise further pharmaceutically acceptable ingredients well known to those skilled in the art. These components include, but are not limited to, pharmaceutically acceptable carriers, diluents, excipients, adjuvants, buffers, preservatives, anti-oxidants, stabilizers, solubilizers, surfactants, and the like.

In some embodiments, the formulation may further comprise other active agents, for example, other therapeutic or prophylactic agents.

Pharmaceutical formulations may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Preferably, especially when the compound is Tasquinimod, it is prepared and administered in a form suitable for oral or parenteral administration, particularly preferred for oral administration. Preferably, especially when the compound is RG2833, it is prepared and administered in a form suitable for oral or parenteral administration, particularly preferred for oral administration. The pharmaceutical formulations may be prepared by any method known in the art of pharmacy, for example by bringing into association the active ingredient with the carrier(s) or excipient(s).

It should be understood that in addition to the ingredients particularly mentioned above, the formulations may include other agents conventional in the art having regard to the type of formulation in question, for example those suitable for oral administration may include flavoring agents.

As used herein the term "therapeutically effective amount" means an amount necessary to at least partly attain the desired response. A therapeutically effective amount of a compound or a pharmaceutically acceptable salt, solvate, hydrate, metabolite or prodrug thereof will depend upon a number of factors including, for example, the age and weight of the subject, the precise condition requiring treatment and its severity, the nature of the formulation, and the route of administration, and will ultimately be at the discretion of the attendant physician or veterinarian.

In one embodiment, the compound for use in the treatment and/or prevention of a kidney disease is administered intravenously or orally, preferably orally.

In one embodiment, the compound for use in the treatment and/or prevention of a kidney disease is a compound of alternative (I) and is administered orally.

In one embodiment, the compound for use in the treatment and/or prevention of a kidney disease is RG2833, wherein RG2833 is administered orally. In one embodiment, the compound for use in the treatment and/or prevention of a kidney disease is a compound of alternative (I) and is administered in a dosage of from 0.1 to 500 mg/d; more preferably from 5 to 450 mg/d, 10 to 400 mg/d, from 15 to 350 mg/d, or from 20 to 300 mg/d; most preferably from 25 to 250 mg/d. In one embodiment, the compound for use in the treatment and/or prevention of a kidney disease is a compound of alternative (I), and is administered orally in a dosage of from 0.1 to 500 mg/d; more preferably from 5 to 450 mg/d, 10 to 400 mg/d, from 15 to 350 mg/d, or from 20 to 300 mg/d; most preferably from 25 to 250 mg/d. In one embodiment, the compound for use in the treatment and/or prevention of a kidney disease is RG2833 and is administered in a dosage of from 0.1 to 500 mg/d; more preferably from 5 to 450 mg/d, 10 to 400 mg/d, from 15 to 350 mg/d, or from 20 to 300 mg/d; most preferably from 25 to 250 mg/d. In one embodiment, the compound for use in the treatment and/or prevention of a kidney disease is RG2833 and is administered orally in a dosage of from 0.1 to 500 mg/d; more preferably from 5 to 450 mg/d, 10 to 400 mg/d, from 15 to 350 mg/d, or from 20 to 300 mg/d; most preferably from 25 to 250 mg/d.

It will be appreciated by one of skill in the art that appropriate dosages of a compound of formula (I), in particular dosages of RG2833, can vary between patients. Determining the optimal dosage will generally involve the balancing of the level of therapeutic benefit against safety risks. Where the dosage of a compound of formula (I), in particular a dosage of RG2833, is provided as a salt, hydrate, solvate, metabolite or prodrug, the amount administered is calculated on the basis of the parent compound and so the actual weight to be used is adjusted proportionately.

In one embodiment, the compound for use in the treatment and/or prevention of a kidney disease is a compound of alternative (II) and is administered orally. In one embodiment, the compound for use in the treatment and/or prevention of a kidney disease is Tasquinimod, wherein Tasquinimod is administered orally. In one embodiment, the compound for use in the treatment and/or prevention of a kidney disease is a compound of alternative (II) and is administered in a dosage from wherein the compound is administered in a dosage of from 0.01 to 5 mg/d; more preferably from 0.05 to 4.5 mg/d, 0.1 to 4 mg/d, from 0.15 to 3 mg/d, or from 0.2 to 2 mg/d; most preferably from 0.2 to 1.5 mg/d. In one embodiment, the compound for use in the treatment and/or prevention of a kidney disease is a compound of alternative (II), and is administered orally in a dosage from wherein the compound is administered in a dosage of from 0.01 to 5 mg/d; more preferably from 0.05 to 4.5 mg/d, 0.1 to 4 mg/d, from 0.15 to 3 mg/d, or from 0.2 to 2 mg/d; most preferably from 0.2 to 1.5 mg/d. In one embodiment, the compound for use in the treatment and/or prevention of a kidney disease is Tasquinimod and is administered in a dosage from wherein the compound is administered in a dosage of from 0.01 to 5 mg/d; more preferably from 0.05 to 4.5 mg/d, 0.1 to 4 mg/d, from 0.15 to 3 mg/d, or from 0.2 to 2 mg/d; most preferably from 0.2 to 1.5 mg/d. In one embodiment, the compound for use in the treatment and/or prevention of a kidney disease is Tasquinimod and is administered orally in a dosage from wherein the compound is administered in a dosage of from 0.01 to 5 mg/d; more preferably from 0.05 to 4.5 mg/d, 0.1 to 4 mg/d, from 0.15 to 3 mg/d, or from 0.2 to 2 mg/d; most preferably from 0.2 to 1.5 mg/d.

It will be appreciated by one of skill in the art that appropriate dosages of a compound of formula (I), in particular dosages of Tasquinimod, can vary between patients. Determining the optimal dosage will generally involve the balancing of the level of therapeutic benefit against safety risks. Where the dosage of a compound of formula (I), in particular a dosage of Tasquinimod, is provided as a salt, hydrate, solvate, metabolite or prodrug, the amount administered is calculated on the basis of the parent compound and so the actual weight to be used is adjusted proportionately.

The above-described aspects of the invention will be further described in the following examples and figures. These are strictly illustrative of particularly preferred embodiments of the invention. The invention should not be construed, however, to be limited to such embodiments.

### EXAMPLES

### Example 1

### Zebrafish strains and husbandry

The zebrafish larva has proven to be an elegant option for drug screens since it combines a high experimental throughput with the advantages of being an *in vivo* model. Optical transparency, expression of fluorescence reporters via genetic engineering, easy and economic maintenance and a 70% genetic homology to humans provide decisive experimental advantages (Howe, K., Clark, M., Torroja, C. et al. (2013) The zebrafish reference genome sequence and its relationship to the human genome. Nature 496, 498-503). A high conservation of the renal filtration apparatus including a strong resemblance of the glomerular filtration barrier make zebrafish larvae very suitable for glomerular research.

Zebrafish stocks were maintained as previously described (Schindler, Maximilian, et al. "Adriamycin does not damage podocytes of zebrafish larvae." Plos one 15.11 (2020): e0242436). Several groups of two males and two females were permanently put together as mating pairs for the screening experiments. These groups were always composed of two individuals of the following zebrafish strains: Tg(*-3.5fabp10a*:gc-eGFP); *mitfaw2*/*w*2;*mpv17a9*/a9 and Tg(-3.5fabp10a:gc-eGFP);Tg(nphs2:GAL4-VP16); Tg(UAS:Eco.nfsB-mCherry); *mitfaw2*/w2; *mpv17a9*/a9 (Xie, Jing, et al. "A novel transgenic zebrafish model for blood-brain and blood-retinal barrier development." BMC developmental biology 10.1 (2010): 1-14; Zhou, Weibin, and Friedhelm Hildebrandt. "Inducible podocyte injury and proteinuria in transgenic zebrafish." Journal of the American Society of Nephrology 23.6 (2012): 1039-1047; Siegerist, Florian, et al. "Acute podocyte injury is not a stimulus for podocytes to migrate along the glomerular basement membrane in zebrafish larvae." Scientific reports 7.1 (2017): 1-12). Larvae from these groups were raised under standard conditions until 4 dpf and transgene expression was checked with a SMZ 18 fluorescence stereomicroscope (Nikon GMBH, Düsseldorf, Germany) under 0.1 mg/ml tricaine anesthesia (MS-222, #E10521, Merck KGaA, Darmstadt, Germany). All experiments adhered with the German animal protection law and were overseen and approved by the "Landesamt für Landwirtschaft, Lebensmittelsicherheit und Fischerei, Rostock" (LALLF M-V) of the federal state of Mecklenburg - Western Pomerania.

### Induction of podocyte injury and drug treatment

Groups of 12 larvae from one clutch were distributed into a 24-well plate (#662160 Greiner, Frickenhausen, Germany). For each clutch, 12 larvae were treated with 0.2% DMSO in E3 as negative control and 12 larvae were treated with 80 µM Metronidazole (MTZ) in DMSO in E3 as injury control and comparison group. The Fraunhofer ScreeningPort IME provided an epigenetic drug library which was used for this screening (Table 1). Compounds from this library were dissolved in MTZ solution in order to perform a co-treatment of larvae with 80 µM MTZ + 100 µM compound in DMSO in E3. The treatment volume was 400 µl for each well with 12 larvae, the wells were sealed with Adhesive Clear PCR Seal (#600208, Biozym Scientific GMBH, Hessisch-Oldendorf) in order to prevent evaporation and plates were incubated at 28.5°C, wrapped in aluminum foil to prevent effects of light. After 24 hours, compounds were washed out in three changes of 0.5 x E3.

### Preparation for imaging

After washout at 5 dpf, E3 in the 12-well plate was replaced with E3 containing 0.4 mg/ml tricaine. Larvae were then individually transferred into wells of a 96-well plate (#644101, Greiner) which was prepared with custom agarose molds. For the molds, 45 µl of 0.7% boiling hot agarose (Biozym LE agarose) were transferred with a multichannel pipette into each well. After 3 min cooldown, a custom 3D printed orientation tool (Wittbrodt, Jonas N., Urban Liebel, and Jochen Gehrig. "Generation of orientation tools for automated zebrafish screening assays using desktop 3D printing." BMC biotechnology 14.1 (2014): 1-6) was inserted into the agarose which produced cavities for orientation. Each larva was transferred with 100 µl tricaine containing E3 into the single wells of the 96-well plate (Row A was always 12x DMSO, Row B was always 12x MTZ, Row C-H were compounds + MTZ) and was then oriented laterally in the molds with a short piece of fishing line glued to a bend injection needle under the stereomicroscope manually with special attention to a flat caudal region.

### Imaging

Image acquisition for the screening was performed with an Imaging Machine (ACQUIRER Imaging GmbH, Heidelberg, Germany). This high-content screening device possesses a white LED array for brightfield images, a LED for fluorescence excitation, a sCMOS (2048 x 2048 pixel) camera, a temperature-controlled incubation chamber (set to 28.5 °C during acquisition), a stationary plate holder and moving optics. Overview images of each laterally oriented larva were obtained with a 2x objective (40% LED intensity, 30 ms integration). For each larva, a region of interest (ROI) at the tail region caudal of the cloaca was selected with the Acquifer PlateViewer (V1.7.1, ACQUIFER) for the 10x objective in order to image the blood flow and vasculature. After creating custom modificated imaging scripts via FIJI (Schindelin, Johannes, et al. "Fiji: an open-source platform for biological-image analysis." Nature methods 9.7 (2012): 676-682), 10 consecutive brightfield frames at a framerate of 33 Hz were acquired (10x objective, 100% LED intensity, 30 ms integration) followed by one frame at 470 nm (10x objective, 50% LED intensity, 50 ms integration). Next, the ROI of each larva was dragged to the glomerular region in the PlateViewer, scripts were centered and one frame of each larval glomerulus at 555 nm was acquired (10x objective, 40% LED intensity, 40 ms integration). The focal plane for both acquisitions was detected with a built-in two-step software autofocus (10x objective, 2x2 binning, 10% LED intensity, 10 ms integration, first step: 31 slices with 50 µm distance, second step: 10 slices with 10 µm slice distance, brightfield for the caudal vasculature, 555 nm for glomerular mCherry).

Following image acquisition, tricaine-containing E3 was washed out two times of each well and replaced by 150 µl E3. The 96-well plate was again sealed and incubated for another 24 h at 28.5°C. At 6 dpf, larvae were again anesthetized with 0.4 mg tricaine, oriented laterally and imaged again in the same way as mentioned before. In this way, two values for the vascular eGFP fluorescence and two values for the glomerular mCherry fluorescence were obtained for each individual larva.

### Data storage and processing

An Acquifer HIVE (ACQUIFER) was used for data storage and processing. Custom written FIJI macros were created and used for image analysis. The macros were written so that a full folder of raw image data of one 96-well plate could be dragged into and directly processed by FIJI.

### Image analysis

### Vascular fluorescence - proteinuria

Vascular eGFP fluorescence was segmented and measured fully automatic via a custom FIJI macro. For each larva, 10 brightfield slices and one slice at 470 nm were acquired at the caudal region. The macro fuses the brightfield slices to a short movie in which the blood flow is clearly observable due to the movement of erythrocytes. After blurring and background subtraction, the standard deviation of this movie is created. This standard deviation shows all areas in which pixel changes occur, which are, in this case, circulating erythrocytes. Subsequent thresholding and size restriction allows the creation of ROIs which distinctively detect areas of blood flow. These ROIs are then used as segmentation masks for the 470 nm image in which the mean fluorescence is measured exclusively in the blood vessels due to prior blood flow detection.

### Glomerular fluorescence - degree of podocyte depletion

Fluorescence of the nphs2-driven podocyte mCherry was segmented and measured fully automatic via FIJI as well. For each larva and each image obtained with 555 nm, the center of mass (x and y coordinate of the brightness-weighted average of all pixel) is determined which is in this case the center of the pronephric glomerulus. A circular custom ROI is created and gets automatically drawn to the center. The ROI for the mCherry signal is always of the same size (70688 pixel with 0.65 µm/pixel ratio) and the mean fluorescence intensity in this ROI is measured for each larva and both time points.

### Data analysis

Both macros create excel files with all necessary data. The macro for the vascular fluorescence additionally creates a subfolder with images of the segmentation masks for the possibility to check the segmentation process. By a build-in exclusion algorithm, larvae in which no vascular blood flow is detected, are not analyzed and values of these larvae will not appear in the excel files but in a text file. Values from these larvae will then also be excluded from the excel files of the glomerular measurement.

For both readouts and each larva there is one mean fluorescence value at 5 dpf right after the treatment and one at 6 dpf. Vascular fluorescence and glomerular fluorescence ratios were calculated for each larva and gaussian distribution was checked with a Kolmogorov-Smirnov test for each readout and each separate plate. Ratios of treatment groups were subsequently analyzed by either ANOVA followed by a Dunnet's multiple comparison test or by a Kruskal-Wallis test followed by Dunn's multiple comparison. The MTZ group on every plate served as a control group, *p* < 0.05 was regarded as statistically significant. Data analysis and graphs were created with GraphPad Prism 9.1.2 (GraphPad Software, San Diego, California USA). Volcano plots were created using VolcaNoseR (10.1101/2020.05.07.082263).

In order to cover two central aspects for the integrity of the larval glomerular filtration barrier, we used the Tg(*-3.5fabp10a:*gc-eGFP); Tg(*nphs2:*GAL4-VP16); Tg(*UAS*:Eco.nfsB-mCherry); *mitfaw2*/w2; *mpv17a9*/a9 strain for all screening experiments. This strain expresses a circulating fusion protein of the vitamin D-binding protein and eGFP (VDP-eGFP) with a size of 78 kDa, which under healthy steady-state conditions cannot pass the glomerular filtration barrier. Impairment of the glomerular filtration barrier directly results in clearance of VDP-eGFP from the vasculature and can be used as a surrogate parameter for proteinuria. Additionally, podocyte-specific expression of the bacterial nitroreductase and mCherry allows microscopic *in vivo* assessment of podocyte presence and podocyte injury induction by immersion treatment (Siegerist, Florian, et al. "Acute podocyte injury is not a stimulus for podocytes to migrate along the glomerular basement membrane in zebrafish larvae." Scientific reports 7.1 (2017): 1-12).

The inventors found that a lateral orientation of the larvae gives perfect microscopic access for the Acquifer Imaging Machine to both readouts, the vascular eGFP fluorescence (degree of Proteinuria) at the tail region and the podocyte mCherry signal (degree of podocyte depletion). Custom agarose molds (Wittbrodt, Jonas N., Urban Liebel, and Jochen Gehrig. "Generation of orientation tools for automated zebrafish screening assays using desktop 3D printing." BMC biotechnology 14.1 (2014): 1-6) were used to generate cavities that allow a swift lateral orientation of 96 larvae per plate.

For the vascular eGFP fluorescence, a region of interest (ROI) was selected with the 10x objective on the basis of an overview image (2x objective) of each larva in a 96-well plate. Using the standard deviation of the pixel change (highest in vasculature with moving blood cells), we were able to reliably and automatically segment the vasculature in zebrafish larvae via FIJI. The custom FIJI analysis script also detects larvae with no blood flow in the caudal vasculature and eliminates these larvae from analysis. This ensures that only viable larvae become part of the evaluation.

For automated segmentation of the mCherry signal in podocytes, a different approach was developed. The segmentation in the brightfield channel is not possible for the glomerulus due to a lack of contrast or pixel value change in the target structure. We directly used the mCherry fluorescence image as a segmentation template and were able to automatically and reliably detect podocytes and measure the mean fluorescence intensity in a defined area.

Variation of transgenic reporter expression levels can vary over clutches of embryos from different founders and therefore possibly increasing heterogeneity in datasets obtained over several weeks. To circumnavigate this, the workflow of this new assay performs two measurements of both readouts for each larva, one at 5 dpf and one at 6 dpf. This generates robust fluorescence ratios for the degree of proteinuria and podocyte condition.

Adding metronidazole (MTZ) to the larval medium causes DNA crosslinking in cells that express the nitroreductase. Other substrates of the nitroreductase such as Nifurpirinol (NFP), however, can also be used. By podocyte-specific nitroreductase expression, this causes an injury exclusively in podocytes. It has been reported that the degree of injury is dependent on MTZ concentration and exposure time (Zhou, Weibin, and Friedhelm Hildebrandt. "Inducible podocyte injury and proteinuria in transgenic zebrafish." Journal of the American Society of Nephrology 23.6 (2012): 1039-1047). The inventors' previous work has shown that a treatment of Tg(*nphs2:*GAL4); Tg(*UAS:Eco.nfsb*-mCherry) larvae at 4 dpf for 48 hours with 80 µM MTZ resulted in a phenotype that resembles mammalian FSGS at 8 dpf (Hansen, Kerrin Ursula Ingeborg, et al. "Prolonged podocyte depletion in larval zebrafish resembles mammalian focal and segmental glomerulosclerosis." The FASEB Journal 34.12 (2020): 15961-15974). In order to avoid feeding and to accelerate the protocol, the MTZ concentration was recalibrated on the Tg(*-3.5fabp10a:*gc-eGFP); Tg(*nphs2:*GAL4-VP16); Tg(*UAS:*Eco.nfsB-mCherry); *mitfaw2*/w2; *mpv17a9*/a9 screening strain.

Larvae were treated with 80 µM 160 µM and 320 µM MTZ for 24 hours. After washout at 5 dpf there was no difference between the DMSO control and the 80 µM group in the mCherry signal. Treatment with 160 µM and 320 µM resulted in a loss of glomerular mCherry and an accumulation of the fluorescence protein in the proximal convoluted tubules.

Imaging of the same larvae after another 24 hours at 6 dpf revealed a loss of mCherry fluorescence in the 80 µM MTZ group compared to the DMSO control. MTZ-induced glomerular injury in the 160 µM and 320 µM lead to a further reduction of mCherry signal intensities, close to a complete loss of podocytes. Podocyte injury or loss leads to proteinuria, in the case of our screening strain to a quantifiable leakage of the VDP-eGFP. After washout of MTZ at 5 dpf, the eGFP signal was not reduced in the 80 µM MTZ group compared to DMSO. Treatment with 160 µM MTZ for 24 h caused a reduction of the vascular eGFP signal and a visible uptake of eGFP in the proximal convoluted tubules. Treatment with 320 µM resulted in a strong glomerular leakage of the VDP-eGFP. At 6 dpf, a clear vascular loss and tubular uptake of the VDP-eGFP was detected in 80 µM MTZ treated larvae compared to the DMSO control. The glomerular leakage in the 160 µM MTZ and 320 µM MTZ group progressed to a state in which a clear vascular signal could not be detected anymore at 6 dpf.

Edema formation is a hallmark of a disrupted filtration barrier in zebrafish (Huang, Jianmin, et al. "A zebrafish model of conditional targeted podocyte ablation and regeneration." Kidney international 83.6 (2013): 1193-1200). Exposure of larvae to 80 µM MTZ for 24 hours from 4-5 dpf induced no edema formation at 5 dpf but slight edema formation at 6 dpf. Slight edema were visible after treatment with 160 µM at 5 dpf, which progressed to severe edema at 6 dpf. Larvae treated with 320 µM MTZ developed severe edema at 5 dpf which even progressed until 6 dpf. Quantifications of the fluorescence intensity ratios by our new screening setup confirmed the prior results. A significant loss of glomerular mCherry and vascular eGFP could be detected in all three MTZ treatment groups by our new assay from 5 to 6 dpf. These results clearly confirm the reliability of this screening method. A treatment with 80 µM MTZ for 24 hours resulted in a quantifiable proteinuric phenotype and podocyte depletion in the timeframe of our screening setup and will be used for all further experiments.

Further details on the methods of Example 1 can also be derived from Schindler, Maximilian, et al. "A Novel High-Content Screening Assay Identified Belinostat as Protective in a FSGS-Like Zebrafish Model." Journal of the American Society of Nephrology 34.12 (2023): 1977-1990.

### Example 2

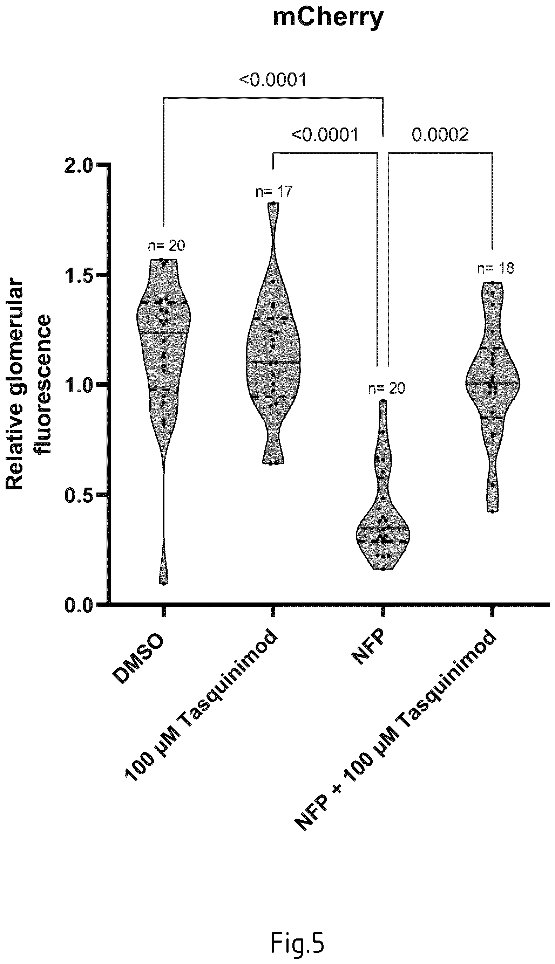

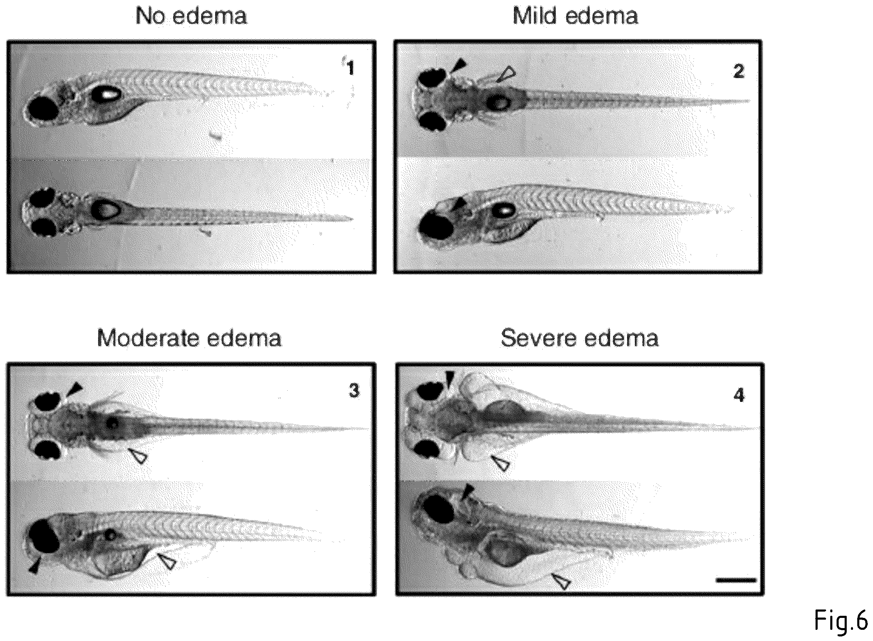

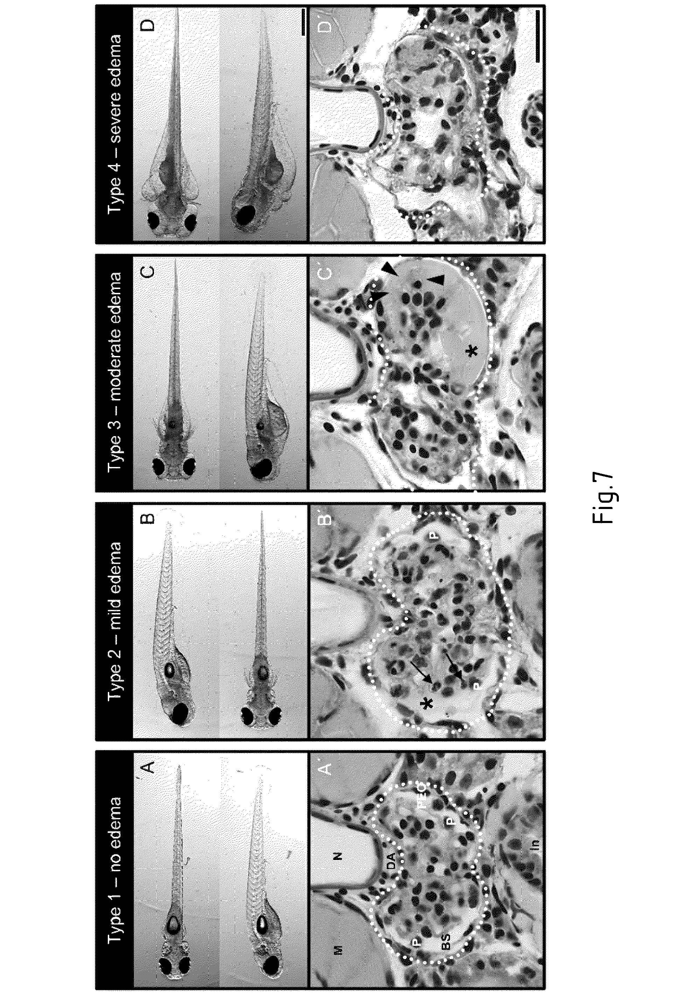

For the assessment of proteinuric edema, groups of 50 *Cherry* larvae (Tg(nphs2:GAL4-VP16); Tg(UAS:Eco.nfsB-mCherry); mitfa^{w2/w2} were treated at 4 days post fertilization (dpf) with either 0.2% DMSO (healthy control group), 50 nM Nifurpirnol (NFP; disease control group) or 50 nM NFP together with either 100 µM Belinostat (positive control group) or 1-100 µM RG2833 for 48 hours. Treatment with NFP causes a specific podocyte injury in *Cherry* larvae and this leads to proteinuric edema (Schindler, Maximilian, et al. "A Novel High-Content Screening Assay Identified Belinostat as Protective in a FSGS-Like Zebrafish Model." Journal of the American Society of Nephrology 34.12 (2023): 1977-1990 and Klawitter, Marianne, et al. "Investigating FSGS-like Injury in Zebrafish Larvae by Nifurpirinol: Efficacy and Molecular Insight." American Journal of Physiology-Renal Physiology (2024)). All compounds were washed out at 6 dpf and and edema formation was assessed in all groups at 6, 7 and 8 dpf according to different categories of severity. The categories range from 1= no edema, 2= mild edema, 3= moderate edema, 4= severe edema. Further categories include malformations of larvae in absence on proteinuric edema (e.g. bend body axis) and dead larvae. The exact presentation of the phenotypes (categories) and the corresponding morphology can be found in Figures 5A and S7 of Schindler, Maximilian, et al. "A Novel High-Content Screening Assay Identified Belinostat as Protective in a FSGS-Like Zebrafish Model." Journal of the American Society of Nephrology 34.12 (2023): 1977-1990, which are reproduced as Figures 6 and 7 in this patent application.

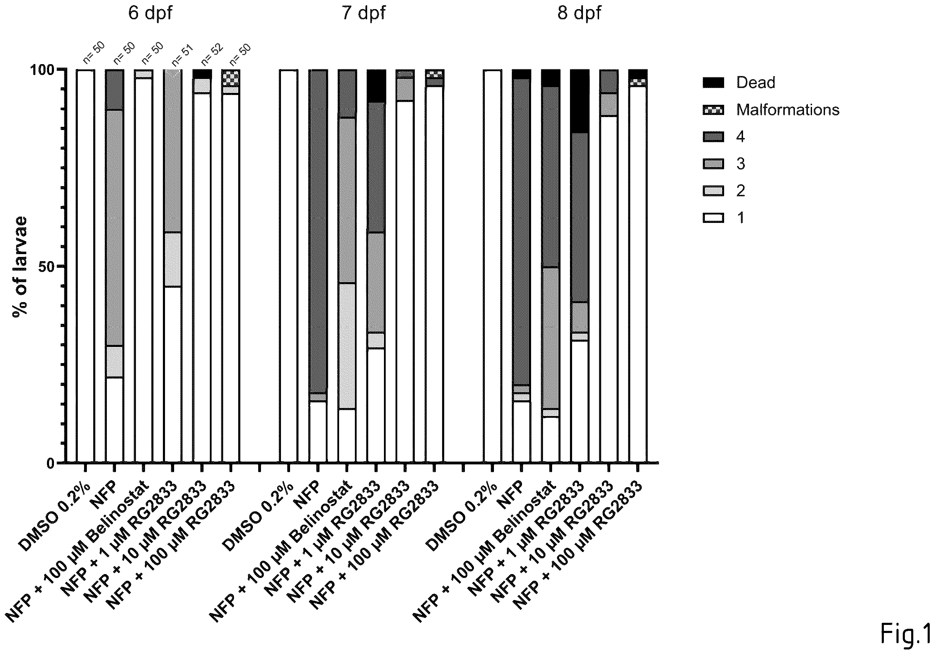

The results of this experiment are depicted in Figure 1.

### Example 3

For the assessment of proteinuric edema, groups of 50 *Cherry* larvae (Tg(nphs2:GAL4-VP16); Tg(UAS:Eco.nfsB-mCherry); mitfa^{w2/w2} were treated at 4 days post fertilization (dpf) with either 0.2% DMSO (healthy control group), 50 nM Nifurpirnol (NFP; disease control group) or 50 nM NFP together with either 100 µM Belinostat (positive control group) or 10-100 µM Tasquinimod for 48 hours. Treatment with NFP causes a specific podocyte injury in *Cherry* larvae and this leads to proteinuric edema (Schindler, Maximilian, et al. "A Novel High-Content Screening Assay Identified Belinostat as Protective in a FSGS-Like Zebrafish Model." Journal of the American Society of Nephrology 34.12 (2023): 1977-1990 and Klawitter, Marianne, et al. "Investigating FSGS-like Injury in Zebrafish Larvae by Nifurpirinol: Efficacy and Molecular Insight." American Journal of Physiology-Renal Physiology (2024)). All compounds were washed out at 6 dpf and and edema formation was assessed in all groups at 6, 7 and 8 dpf according to different categories of severity. The categories range from 1= no edema, 2= mild edema, 3= moderate edema, 4= severe edema. Further categories include malformations of larvae in absence on proteinuric edema (e.g. bend body axis) and dead larvae. The exact presentation of the phenotypes (categories) and the corresponding morphology can be found in Figures 5A and S7 of Schindler, Maximilian, et al. "A Novel High-Content Screening Assay Identified Belinostat as Protective in a FSGS-Like Zebrafish Model." Journal of the American Society of Nephrology 34.12 (2023): 1977-1990, which are reproduced as Figures 6 and 7 in this patent application.

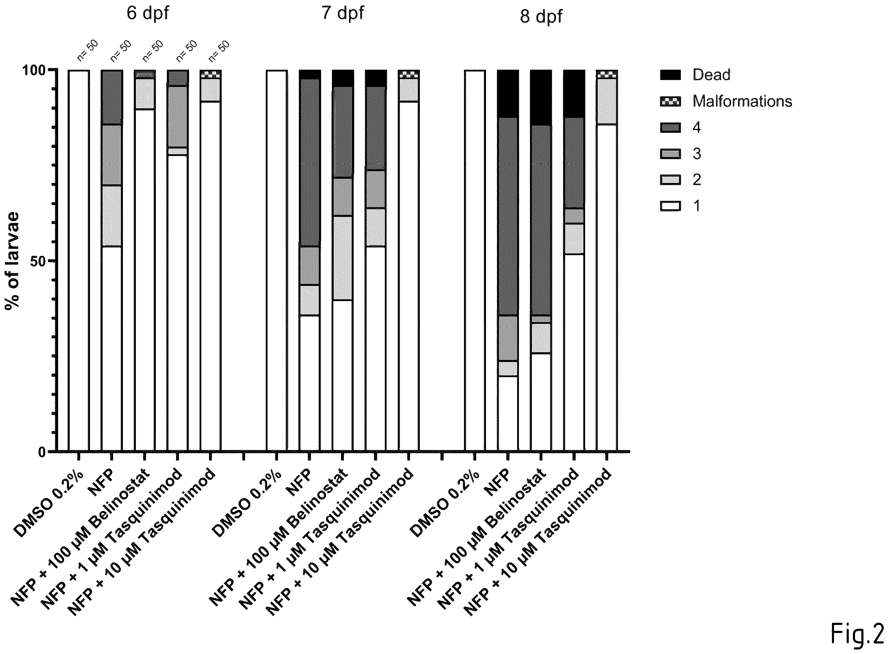

The results of this experiment are depicted in Figure 2.

### Example 4

In a pre-treatment experiment, groups of 50 *Cherry* larvae were treated with 0.1% DMSO or 100 µM Tasquinimod for 24 hours. After washout, the groups were either treated with 0.1% DMSO or NFP for 48 hours until 7 dpf. After washout, edema formation was assessed in all groups at 7 and 8 dpf according to different categories of severity. The categories range from 1= no edema, 2= mild edema, 3= moderate edema, 4= severe edema. Further categories include malformations of larvae in absence on proteinuric edema (e.g. bend body axis) and dead larvae. The exact presentation of the phenotypes (categories) and the corresponding morphology can be found in Figures 5A and S7 of Schindler, Maximilian, et al. "A Novel High-Content Screening Assay Identified Belinostat as Protective in a FSGS-Like Zebrafish Model." Journal of the American Society of Nephrology 34.12 (2023): 1977-1990, which are reproduced as Figures 6 and 7 in this patent application.

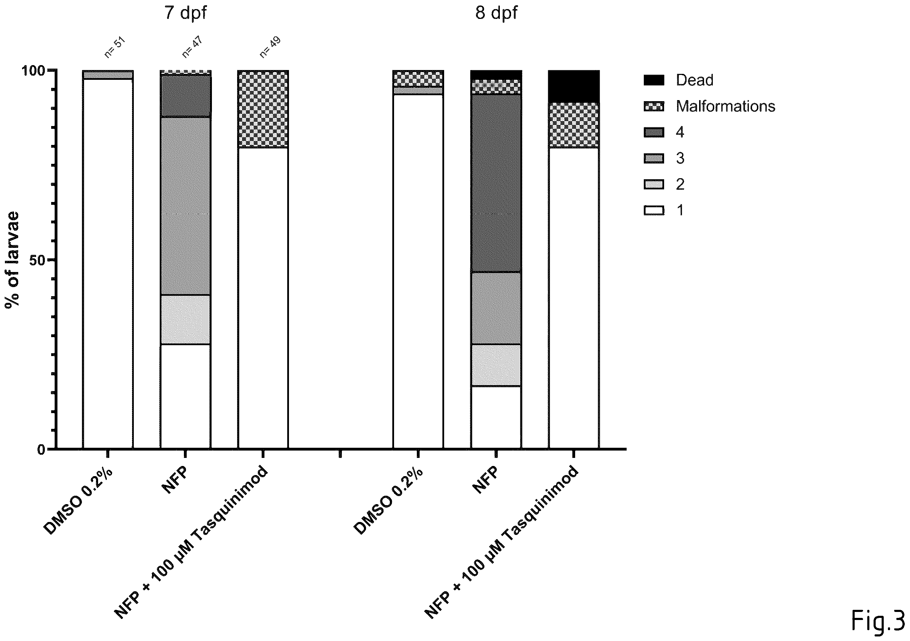

The results of this experiment are depicted in Figure 3.

### Example 5

In a pre-treatment experiment, groups of 50 *Cherry* larvae were treated with either 0.1% DMSO or 100 µM RG2833 for 24 hours. After washout, the groups were either treated with 0.1% DMSO or NFP for 48 hours until 7 dpf. After washout, edema formation was assessed in all groups at 7 and 8 dpf according to different categories of severity. The categories range from 1= no edema, 2= mild edema, 3= moderate edema, 4= severe edema. Further categories include malformations of larvae in absence on proteinuric edema (e.g. bend body axis) and dead larvae. The exact presentation of the phenotypes (categories) and the corresponding morphology can be found in Figures 5A and S7 of Schindler, Maximilian, et al. "A Novel High-Content Screening Assay Identified Belinostat as Protective in a FSGS-Like Zebrafish Model." Journal of the American Society of Nephrology 34.12 (2023): 1977-1990, which are reproduced as Figures 6 and 7 in this patent application.

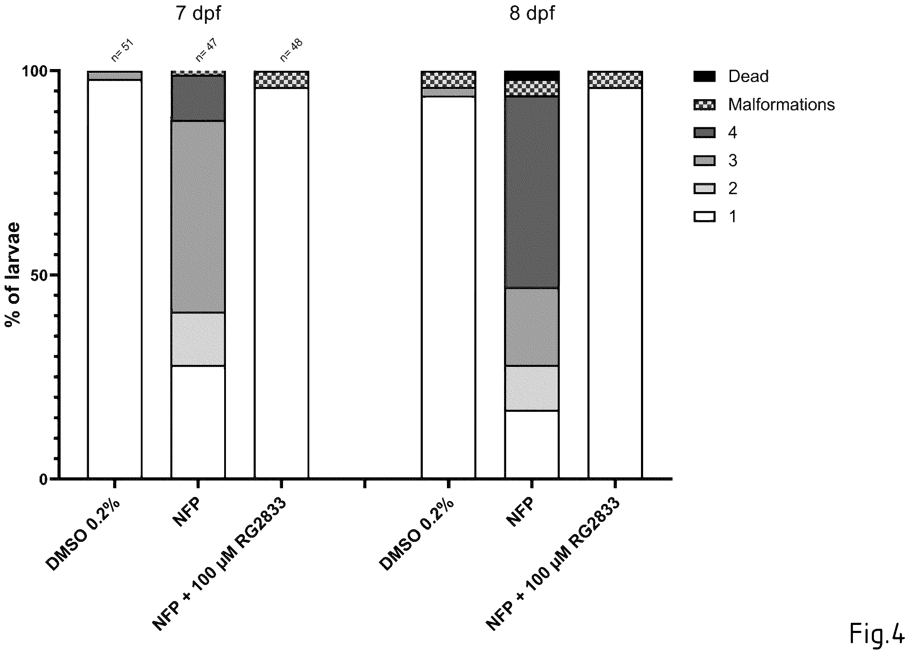

The results of this experiment are depicted in Figure 4.

### Example 6

Groups of *24 ScreeFi* larvae(Tg(-*3.5fabp10a:*gc-eGFP); Tg(*nphs2:*GAL4-VP16); Tg(*UAS*:Eco.nfsB-mCherry); *mitfa*^{*w2*/w2}) were treated with either 0.2% DMSO, 100 µM Tasquinimod, 50 nM NFP or 50 nM NFP together with 100 µM Tasquinimod for 24 hours from 4-5 dpf. After washout, larvae were anesthetized, transferred to a 96-well plate prepared with custom agarose molds and orientated laterally. The mCherry signal in the pronephric glomerulus was imaged with the Acquifer Imaging Machine (10x objective) at 5 dpf. After imaging, the anesthetic was washed out and larvae were incubated for another 24 hours in E3 medium. At 6 dpf, the glomerular mCherry signal of the same larvae was acquired again. A custom macro code written in the IJ1 language (repository for the code: https://github.com/MaximilianSchindler/ImageJ-macros) automatically detects the mCherry fluorescence and creates a region of interest of 150x150 pixels (whole image resolution: 2048x2048) around the glomerulus. The mean mCherry fluorescence of the larval glomerulus is then measured within this area at both timepoints. Following this, the individual mCherry intensity ratio (raw grayscale value at 6 dpf / raw grayscale value at 5 dpf) was determined and used as input for statistics. Thus, ratios <1 describe a loss of fluorescence, a ratio of 1 describes no changes and ratios >1 depict an increase of fluorescence between 5 and 6 dpf. After checking gaussian distribution, groups were compared with a Kruskal-Wallis test followed by a Dunn's multiple comparison test. p-values of <0.05 were considered stastically significant.

The results of this experiment are depicted in Figure 5.

### BRIEF DESCRIPTION OF THE DRAWINGS

Figure 1 shows the effects of RG2833 on the development of proteinuric edema (category 1: no edema, category 2: mild edema, category 3: moderate edema, category 4: severe edema). *Cherry* larvae treated with DMSO group (healthy control) did not develop proteinuric edema. In contrast, 50 nM NFP treatment caused the development of proteinuric edema in >75% at 6 dpf and >80% of larvae at 7 and 8 dpf. Co-treatment of *Cherry* larvae with 50 nM NFP and 100 µM Belinostat (positive control) caused a prevention of edema formation at 6 dpf. The protective effect of Belinostat decreased as >80% of larvae in this group developed proteinuric edema at 7 and 8 dpf. Co-treatment with 50 nM NFP and RG2833 completely prevented edema formation in the groups that received 10 or 100 µM RG2833. In contrast to the Belinostat group, the protective effect of RG2833 prevailed and larvae did not develop proteinuric edema at all.
Figure 2 shows the effects of Tasquinimod on the development of proteinuric edema (category 1: no edema, category 2: mild edema, category 3: moderate edema, category 4: severe edema). Cherry larvae treated with DMSO group (healthy control) did not develop proteinuric edema. In contrast, 50 nM NFP treatment caused the development of proteinuric edema in >45% at 6 dpf and >75% of larvae at 7 and 8 dpf, respectively. Co-treatment of Cherry larvae with 50 nM NFP and 100 µM Belinostat (positive control) caused a prevention of edema formation at 6 dpf. The protective effect of Belinostat decreased as >60% and >70% of larvae in this group developed proteinuric edema at 7 and 8 dpf, respectively. Co-treatment of Cherry larvae with 50 nM NFP and 1 or 10 µM Tasquinimod showed a concentration dependent protective effect. In contrast to the Belinostat group, the protective effect of Tasquinimod prevailed after washout over time.
Figure 3 shows the effects of a pre-treatment with Tasquinimod on the development of proteinuric edema (category 1: no edema, category 2: mild edema, category 3: moderate edema, category 4: severe edema). Cherry larvae were treated with DMSO or 100 µM Tasquinimod for 24 hours from 4-5 dpf before induction of podocyte injury. Subsequent treatment with 50 nM NFP from 6-7 dpf or DMSO as healthy control and edema assessment at 7 and 8 dpf revealed that the pre-treatment with 100 µM Tasquinimod completely prevented edema formation in contrast to the 50 nM NFP group.
Figure 4 shows the effects of a pre-treatment with RG2833 on the development of proteinuric edema (category 1: no edema, category 2: mild edema, category 3: moderate edema, category 4: severe edema). *Cherry* larvae were treated with DMSO or 100 µM RG2833 for 24 hours from 4-5 dpf before induction of podocyte injury. Subsequent treatment with 50 nM NFP from 6-7 dpf or DMSO as healthy control and edema assessment at 7 and 8 dpf revealed that the pre-treatment with 100 µM RG2833 completely prevented edema formation in contrast to the NFP group.
Figure 5 shows the effects of Tasquinimod on the glomerular mCherry fluorescence. ScreeFi larvae were treated with DMSO, 100 µM Tasquinimod, 50 nM NFP or 50 nM NFP + 100 µM Tasquinimod for 24 hours from 4-5 dpf. The glomerular mCherry signal was acquired at 5 and 6 dpf and the glomerular mCherry fluorescence ratio was determined. The DMSO group served as healthy control. Treatment with 100 µM Tasquinimod alone showed no effect compared to the DMSO group. 50 nM NFP treatment caused a significant decrease of glomerular fluorescence due to podocyte injury. The co-treatment of Tasquinimod with NFP significantly decreased loss of fluorescence compared to the 50 nM NFP group. Tasquinimod mitigated the podocyte injury and the subsequent loss of the mCherry signal in podocytes.
Figure 6 depicts the reproduced Figure 5A of Schindler, Maximilian, et al. "A Novel High-Content Screening Assay Identified Belinostat as Protective in a FSGS-Like Zebrafish Model." Journal of the American Society of Nephrology 34.12 (2023): 1977-1990. This figure shows the exact presentation of the edema phenotypes (categories) and the corresponding morphology. As drug validation experiment, Cherry larvae were treated with different concentrations of belinostat in conjunction with MTZ or post-MTZ treatment. Depending on the progression of podocyte injury, larvae developed different degrees of edema: 1: no edema, 2: mild edema, 3: moderate edema, and 4: severe edema. Black arrowheads show periocular edema, and blank arrowheads depict abdominal edema.
Figure 7 depicts the reproduced Figure S7 of Schindler, Maximilian, et al. "A Novel High-Content Screening Assay Identified Belinostat as Protective in a FSGS-Like Zebrafish Model." Journal of the American Society of Nephrology 34.12 (2023): 1977-1990. MTZ induced specific podocyte depletion results in development of periocular and abdominal edema of different severities are shown (A-D). H&E staining of 4 µm transversal plastic sections show a healthy glomerular morphology of type 1 larvae without edema including podocytes on the visceral leaf of the glomerulus (A'). Larvae that display type 2 edema show pathologic alterations in the glomerulus such as apoptotic nuclei (arrows) and a leaky filtration barrier shown by eosin positive protein casts in the Bowman's space (asterisk) (B'). The glomerular morphology of larvae with type 3 edema displays a strongly affected phenotype. A clear identification of podocytes on the visceral leaf is not possible anymore and there appears to be naked GBM on one side of the glomerulus (arrowheads). Additionally, a strong accumulation of protein casts in the Bowman's space (asterisk) is visible, indicating massive proteinuria (C'). Glomeruli of larvae with type 4 edema present severe glomerular injury, podocyte depletion has led to a complete disintegration of the glomerular morphology (D'). Dashed line: Glomerular outline; BS: Bowman's space; DA: Dorsal aorta; In: Intestine; M: Myotome; N: Notochord; P: Podocyte; PEC: Parietal epithelial cell. Scale bar in (D): 500 µm, in (D'): 20 µm.

## Claims

1. A compound for use in the treatment and/or prevention of a kidney disease, wherein the compound has the general formula (I): 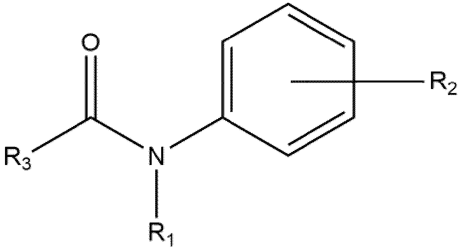 wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₂ is selected from the group consisting of -NH₂, C₁-C₃-haloalkyl, C₁-C₃-alkyl, C₁-C₃-alkoxy, C₁-C₃-haloalkoxy, C₃-C₆-cycloalkyl, halogen, -OH, -SH, -CN, -CHO, -NH(C₁-C₃-alkyl), and -N(C₁-C₃-alkyl)₂;
R₃ is selected from the group consisting of 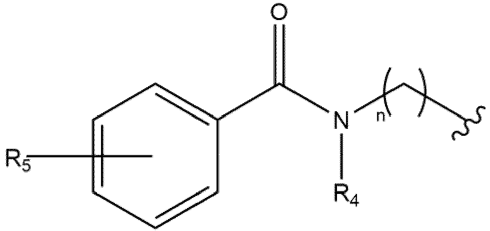 and 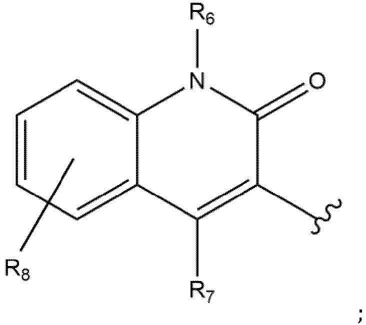
R₄ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
n is 2, 3, 4, 5, 6, or 7;
R₅ is selected from the group consisting of C₁-C₃-alkyl, C₁-C₃-haloalkyl, C₁-C₃-alkoxy, C₃-C₆-cycloalkyl, C₂-C₄-alkenyl, C₂-C₄-alkynyl, halogen, -OH, -SH, -CN, -CHO, -COOH, -NH₂, -CONH₂, -CSNH₂, -NH(C₁-C₃-alkyl), and -N(C₁-C₃-alkyl)₂;
R₆ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₇ is selected from the group consisting of -OH, -CN, -SH, -CHO, COOH, -NH₂, -NH(C₁-C₃-alkyl), -N(C₁-C₃alkyl)₂, C₁-C₃-alkoxy, -O-C=O-C₁-C₃alkyl, and C₁-C₃-hydroxyalkyl; and
R₈ is selected from the group constisting of C₁-C₃-alkoxy, C₁-C₃-alkyl, C₁-C₃-hydroxyalkyl, C₁-C₃-haloalkyl, C₂-C₄-alkenyl, C₂-C₄-alkynyl, -OH, -SH, -CN, -CHO, - COOH, NH₂, -CONH₂, -CSNH₂, -NH(C₁-C₃-alkyl), and -N(C₁-C₃alkyl)₂;
or a salt, hydrate, solvate, metabolite, or prodrug thereof.

2. A compound for use according to claim 1, or a salt, hydrate, solvate, metabolite, or prodrug thereof,
wherein
Ra is

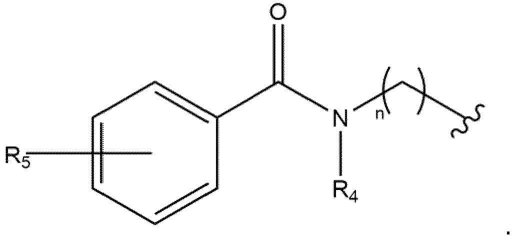

3. A compound for use according to claim 1 or 2, or a salt, hydrate, solvate, metabolite, or prodrug thereof,
wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₂ is selected from the group consisting of -NH₂, C₁-C₃-haloalkyl, C₁-C₃-alkoxy, C₁-C₃-haloalkoxy, halogen, -OH, -SH, -CN, -CHO, -NH(C₁-C₃-alkyl), and -N(C₁-C₃alkyl)₂;
R₄ is selected from the group consisting of hydrogen, C₁-C₃-alkyl, C₁-C₃-haloalkyl, C₃-C₆-cycloalkyl, and C₁-C₃-alkoxy;
n is 4, 5, or 6, preferably 5; and
R₅ is selected from the group consisting of C₁-C₃-alkyl, C₁-C₃-haloalkyl, C₁-C₃-alkoxy, C₃-C₆-cycloalkyl, C₂-C₄-alkenyl, C₂-C₄-alkynyl, halogen, -OH, -SH, -CN, -CHO, -COOH, -NH₂, -CONH₂, -CSNH₂, -NH(C₁-C₃-alkyl), and -N(C₁-C₃alkyl)₂.

4. A compound for use according to any one of claims 1, 2 or 3, or a salt, hydrate, solvate, metabolite, or prodrug thereof,
wherein
R₁ is selected from the group consisting of hydrogen, C₁-C₃-alkyl, and C₃-C₆-cycloalkyl;
R₂ is selected from the group consisting of -NH₂, C₁-C₃-haloalkyl, C₁-C₃-alkoxy, halogen, -OH, -SH, -CN, -CHO, -NH(C₁-C₃-alkyl), and -N(C₁-C₃alkyl)₂;
R₄ is selected from the group consisting of hydrogen, C₁-C₃-alkyl, and C₃-C₆-cycloalkyl;
n is 5; and
R₅ is selected from the group consisting of C₁-C₃-alkyl, C₁-C₃-haloalkyl, C₁-C₃-alkoxy, C₃-C₆-cycloalkyl, C₂-C₄-alkenyl, and C₂-C₄-alkynyl.

5. A compound for use according to any one of claims 1, 2, 3 or 4, or a salt, hydrate, solvate, metabolite, or prodrug thereof,
wherein the compound is

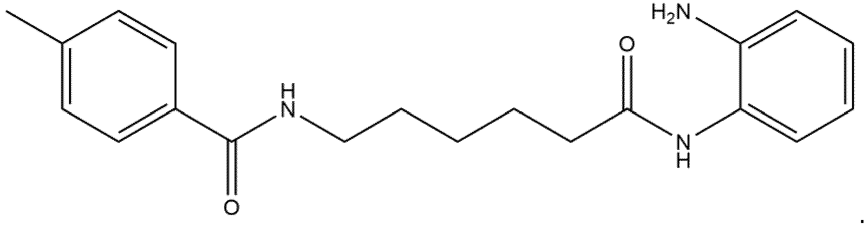

6. A compound for use according to claim 5, wherein the metabolite is selected from the group consisting of a benzimidazole, a glucuronide, and products of amide hydrolysis including o-phenylenediamine, acetylated phenylenediamine, and acids.

7. A compound for use according to claim 1, or a salt, hydrate, solvate, metabolite, or prodrug thereof,
wherein
Ra is

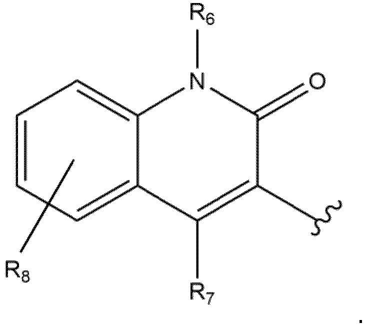

8. A compound for use according to claim 1 or 7, or a salt, hydrate, solvate, metabolite, or prodrug thereof,
wherein
R₁ is selected from the group consisting of hydrogen, C₁-C₃-alkyl, and C₃-C₆-cycloalkyl;
R₂ is selected from the group consisting of -NH₂, C₁-C₃-haloalkyl, preferably -CF₃, C₁-C₃-alkyl,
C₁-C₃-alkoxy, C₁-C₃-haloalkoxy, C₃-C₆-cycloalkyl, halogen, -OH, -SH, -CN, -CHO, - NH(C₁-C₃-alkyl) and
-N(C₁-C₃alkyl)₂;
R₆ is selected from the group consisting of hydrogen, C₁-C₃-alkyl, C₁-C₃-haloalkyl, C₃-C₆-cycloalkyl, and C₁-C₃-alkoxy;
R₇ is selected from the group consisting of -OH, -CN, -SH, -CHO, COOH, -NH₂, - NH(C₁-C₂-alkyl), -N(C₁-C₂-alkyl)₂, C₁-C₂-alkoxy, -O-C=O-C₁-C₃alkyl, and C₁-C₂-hydroxyalkyl; and
R₈ is selected from the group consisting of C₁-C₃-alkoxy, C₁-C₃-alkyl, C₁-C₃-hydroxyalkyl, C₁-C₃-haloalkyl, -OH, -SH, -CN, -CHO, -COOH, -NH₂, -CONH₂, -CSNH₂, - NH(C₁-C₃-alkyl), and -N(C₁-C₃alkyl)₂.

9. A compound for use according any one of claims 1, 7 or 8, or a salt, hydrate, solvate, metabolite, or prodrug thereof,
wherein
R₁ is selected from the group consisting of hydrogen, C₁-C₃-alkyl, C₁-C₃-haloalkyl, C₃-C₆-cycloalkyl, C₂-C₄-alkenyl, C₂-C₄-alkynyl, and C₁-C₃-alkoxy;
R₂ is selected from the group consisting of -NH₂, C₁-C₃-haloalkyl, preferably -CF₃, C₁-C₃-alkyl,
C₁-C₃-alkoxy, C₃-C₆-cycloalkyl, halogen, -OH, -SH, -CN, -CHO, -NH(C₁-C₃-alkyl), and -N(C₁-C₃alkyl)₂;
R₆ is selected from the group consisting of hydrogen, C₁-C₃-alkyl, and C₃-C₆-cycloalkyl;
R₇ is selected from the group consisting of -OH, -CN, -SH, -CHO, COOH, -NH₂, -NH-CH₃, -N(CH₃)₂, -OCH₃, -O-C=O-CH₂-CH₃, and -CH₂-OH; and
R₈ is selected from the group consisting of C₁-C₂-alkoxy, C₁-C₂-alkyl, C₁-C₂-hydroxyalkyl, C₁-C₂-haloalkyl, -OH, -SH, -CN, -CHO, -COOH, -NH₂, -CONH₂, - CSNH₂, -NH(C₁-C₂-alkyl), and -N(C₁-C₂-alkyl)₂.

10. A compound for use according to any one of claims 1, 7, 8 or 9, or a salt, hydrate, solvate, metabolite, or prodrug thereof,
wherein the compound is

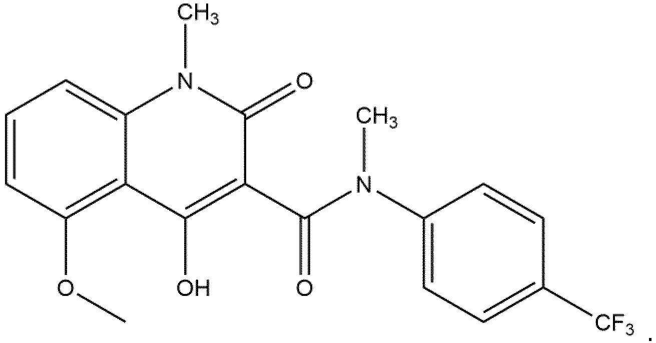

11. A compound for use according to claim 10,
wherein the metabolite is selected from the group consisting of

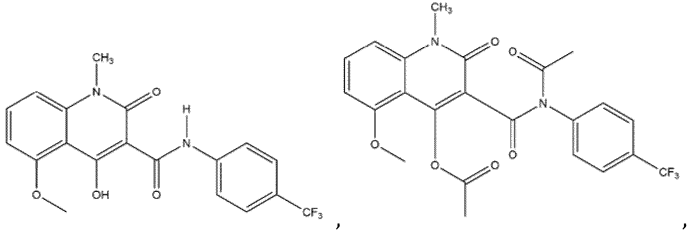

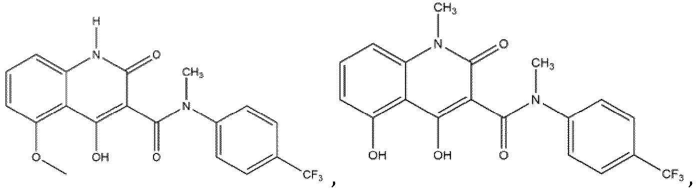

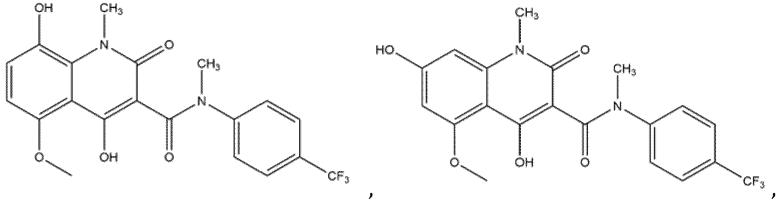

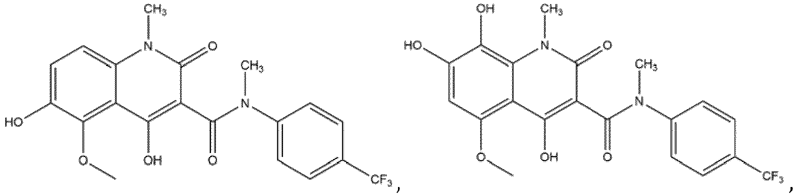

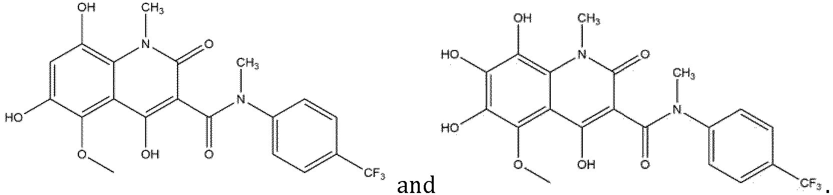

12. The compound for use according to any one of the preceding claims, wherein the compound is administered intravenously or orally, preferably orally.

13. The compound for use according to any one of claims 1, 2, 3, 4, 5, 6 or 12, wherein the compound is administered in a dosage of from 0.1 to 500 mg/d; more preferably from 5 to 450 mg/d, 10 to 400 mg/d, from 15 to 350 mg/d, or from 20 to 300 mg/d; most preferably from 25 to 250 mg/d.

14. The compound for use according to any one of claims 1, 7, 8, 9, 10, 11 or 12,
wherein the compound is administered in a dosage of from 0.01 to 5 mg/d; more preferably from 0.05 to 4.5 mg/d, 0.1 to 4 mg/d, from 0.15 to 3 mg/d, or from 0.2 to 2 mg/d; most preferably from 0.2 to 1.5 mg/d.

15. The compound for use according to any one of the preceding claims, wherein the kidney disease is an acute or chronic kidney disease.

16. The compound for use according to any one of the preceding claims, wherein the kidney disease is a kidney disease associated with podocyte injury and/or podocyte foot process effacement.

17. The compound for use according to any one of the preceding claims, wherein the kidney disease is
(i) a podocytopathy; in particular a podocytopathy selected from the group consisting of diffuse mesangial sclerosis, focal segmental glomerulosclerosis, minimal change disease, and collapsing glomerulopathy;
(ii) a genetic kidney disease associated with one or more genes located in the podocyte;
(iii) is selected from the group consisting of secondary focal and segmental glomerulosclerosis, hypertension-induced nephropathy, diabetic nephropathy, minimal change disease, and membranous nephropathy; and/or
(iv) is selected from the group consisting of diabetic nephropathy, secondary focal and segmental glomerulosclerosis, and minimal change disease.
